# EUROPEAN PATENT SPECIFICATION

(11) **EP 2 928 493 B1**
(45) Date of publication and mention of the grant of the patent: **07.08.2019**
(21) Application number: 13860021.8
(22) Date of filing: 03.12.2013
(51) Int. Cl.: A61K 39/104

(54) **FUSION PROTEINS FOR USE AS IMMUNOGENIC ENHANCERS FOR INDUCING ANTIGEN-SPECIFIC T CELL RESPONSES**
FUSIONSPROTEINE ZUR VERWENDUNG ALS IMMUNOGENE VERSTÄRKER ZUR INDUKTION VON ANTIGENSPEZIFISCHEN T-ZELL-ANTWORTEN
PROTÉINES HYBRIDES UTILISÉES EN TANT QU'ACTIVATEURS IMMUNOGÈNES POUR INDUIRE DES RÉPONSES LYMPHOCYTAIRES T SPÉCIFIQUES DE L'ANTIGÈNE

(30) Priority: 05.12.2012 US 201261733879 P
(43) Date of publication of application: 14.10.2015
(73) Proprietor: TheVax Genetics Vaccine Co., Ltd., Taipei City, Taiwan 10685 (CN)
(72) Inventor: WU, Chia-Mao, Zhubei City Shinchu County 302 Taiwan (TW); CHANG, Hsiu-Kang, Taipei City 10685 Taiwan (TW)
(74) Representative: Gill, Siân Victoria
(86) International application number: PCT/US2013/072753
(87) International publication number: WO 2014/089009

(56) References cited:
- WO-A1-00/49041
- WO-A2-01/29233
- WO-A2-99/45127
- WO-A2-03/085085
- WO-A2-2004/087196
- US-A1- 2003 158 102
- US-A1- 2006 217 298
- US-A1- 2008 132 450
- US-A1- 2009 018 079
- US-A1- 2009 214 570
- US-A1- 2011 028 403
- US-A1- 2012 021 996
- US-A1- 2012 065 385
- US-A1- 2012 213 811
- US-A1- 2012 263 748
- TAKEMOTO ET AL.: 'Enhanced generation of cytotoxic T lymphocytes by heat shock protein 70 fusion proteins harboring both CD 6(.+) T cell and CD 4(+) T cell epitopes.' MOL PHARM. vol. 7, no. 5, 2010, pages 1715 - 1723, XP 055255754
- CHIANG ET AL.: 'Novel Synthetic Bipartite Carrier Protein for Developing Glycotope-Based Vaccines.' VACCINE EPUB vol. 30, no. 52, 22 October 2012, pages 7573 - 7581, XP 055255756
- EGERER ET AL.: 'Secreted antiviral entry inhibitory (SAVE) peptides for gene therapy of HIV infection.' MOL THER. vol. 19, no. 7, 2011, pages 1236 - 1244, XP 055137012
- PESU ET AL.: 'Proprotein convertase furin is preferentially expressed in T helper 1 cells and regulates interferon gamma.' BLOOD vol. 108, no. 3, 2006, pages 983 - 985, XP055255758
- EVANS ET AL.: 'Crystal structure of a soluble CD 28-Fab complex.' NAT LMMUNOL. vol. 6, no. 3, 2005, pages 271 - 279, XP 055255759
- BOOMER ET AL.: 'An Enigmatic Tail of CD 28 Signaling.' COLD SPRING HARB PERSPECT BIOL. vol. 2, no. 8, 2010, page 12, XP 055184340
- RUECKERT ET AL.: 'Vaccines: from empirical development to rational design.' PLOS PATHOG. vol. 8, no. 11, 08 November 2012, pages 3 - 4, XP055255763
- CAMINSCHI. ET AL.: 'Boosting antibody responses by targeting antigens to dendritic cells.' TRENDS IMMUNOL TRENDS IMMUNOL. vol. 33, no. 2, February 2012, pages 71 - 77, XP002715556
- PARK ET AL.: 'Enhanced Induction of T Cell Immunity Using Dendritic Cells Pulsed with HIV Tat and HCMV-pp65 Fusion Protein In Vitro.' IMMUNE NETW. vol. 11, no. 3, 2011, pages 182 - 189, XP055255765
- LIU ET AL.: 'A novel therapeutic fusion protein vaccine by two different families of heat shock proteins linked with HPV16 E7 generates potent antitumor immunity and antiangiogenesis.' VACCINE vol. 26, no. 10, 2008, pages 1387 - 1396, XP022492633
- PAWARIA ET AL.: 'CD 91-dependent programming of T-helper cell responses following heat shock protein immunization.' NAT COMMUN. vol. 2, no. 521, 2011, XP055255768
- BINDER ET AL.: 'CD 91-Dependent Modulation of Immune Responses by Heat Shock Proteins: A Role in Autoimmunity.' AUTOIMMUNE DIS. 2012, XP055255769
- CRUZ ET AL.: 'Enhancing immunogenicity and cross-reactivity of HIV-1 antigens by in vivo targeting to dendritic cells.' NANOMEDICINE vol. 7, no. 10, October 2012, LOND, pages 1591 - 1610, XP008179318

### SEQUENCE LISTING

<110> TheVax Genetics Vaccine Co., Ltd WU, CHIA-MAO CHANG, HSIU-KANG
<120> FUSION PROTEINS AS IMMUNOGEN ENHANCERS FOR ENHANCING ANTIGEN-SPECIFIC T CELL RESPONSES
<130> 10021-00001
<150> US/61733879
   <151> 2012-12-05
<160> 32
<170> PatentIn version 3.5
<210> 1
   <211> 28
   <212> PRT
   <213> Artificial Sequence
<220>
   <223> hCD28 Core
<400> 1
   Thr Asp Ile Tyr Phe Cys Lys Ile Glu Val Met Tyr Pro Pro Pro Tyr
   1               5                   10                  15
   Leu Asp Asn Glu Lys Ser Asn Gly Thr Ile Ile His
               20                  25
<210> 2
   <211> 53
   <212> PRT
   <213> Artificial Sequence
<220>
   <223> hCD28 Maximum
<400> 2
   Asn Cys Asp Gly Lys Leu Gly Asn Glu Ser Val Thr Phe Tyr Leu Gln
   1               5                   10                  15
   Asn Leu Tyr Val Asn Gln Thr Asp Ile Tyr Phe Cys Lys Ile Glu Val
               20                  25                  30
   Met Tyr Pro Pro Pro Tyr Leu Asp Asn Glu Lys Ser Asn Gly Thr Ile
           35                  40                  45
   Ile His Val Lys Gly
       50
<210> 3
   <211> 34
   <212> PRT
   <213> Artificial Sequence
<220>
   <223> PEt Core
<400> 3
   Gly Trp Glu Gln Leu Glu Gln Cys Gly Tyr Pro Val Gln Arg Leu Val
   1               5                   10                  15
   Ala Leu Tyr Leu Ala Ala Arg Leu Ser Trp Asn Gln Val Asp Gln Val
               20                  25                  30
   Ile Arg
<210> 4
   <211> 112
   <212> PRT
   <213> Artificial Sequence
<220>
   <223> PEt Maximum
<400> 4
   Gly Gly Ser Leu Ala Ala Leu Thr Ala His Gln Ala Cys His Leu Pro
   1               5                   10                  15
   Leu Glu Thr Phe Thr Arg His Arg Gln Pro Arg Gly Trp Glu Gln Leu
               20                  25                  30
   Glu Gln Cys Gly Tyr Pro Val Gln Arg Leu Val Ala Leu Tyr Leu Ala
           35                  40                  45
   Ala Arg Leu Ser Trp Asn Gln Val Asp Gln Val Ile Arg Asn Ala Leu
       50                  55                  60
   Ala Ser Pro Gly Ser Gly Gly Asp Leu Gly Glu Ala Ile Arg Glu Gln
   65                  70                  75                  80
   Pro Glu Gln Ala Arg Leu Ala Leu Thr Leu Ala Ala Ala Glu Ser Glu
                   85                  90                  95
   Arg Phe Val Arg Gln Gly Thr Gly Asn Asp Glu Ala Gly Ala Ala Asn
               100                 105                 110
<210> 5
   <211> 104
   <212> PRT
   <213> Artificial Sequence
<220>
   <223> RAP1 Minimum
<400> 5
   Ala Glu Phe Glu Glu Pro Arg Val Ile Asp Leu Trp Asp Leu Ala Gln
   1               5                   10                  15
   Ser Ala Asn Leu Thr Asp Lys Glu Leu Glu Ala Phe Arg Glu Glu Leu
               20                  25                  30
   Lys His Phe Glu Ala Lys Ile Glu Lys His Asn His Tyr Gln Lys Gln
           35                  40                  45
   Leu Glu Ile Ala His Glu Lys Leu Arg His Ala Glu Ser Val Gly Asp
       50                  55                  60
   Gly Glu Arg Val Ser Arg Ser Arg Glu Lys His Ala Leu Leu Glu Gly
   65                  70                  75                  80
   Arg Thr Lys Glu Leu Gly Tyr Thr Val Lys Lys His Leu Gln Asp Leu
                   85                  90                  95
   Ser Gly Arg Ile Ser Arg Ala Arg
               100
<210> 6
   <211> 153
   <212> PRT
   <213> Artificial Sequence
<220>
   <223> A2M Minimum
<400> 6
   Val Tyr Leu Gln Thr Ser Leu Lys Tyr Asn Ile Leu Pro Glu Lys Glu
   1               5                   10                  15
   Glu Phe Pro Phe Ala Leu Gly Val Gln Thr Leu Pro Gln Thr Cys Asp
               20                  25                  30
   Glu Pro Lys Ala His Thr Ser Phe Gln Ile Ser Leu Ser Val Ser Tyr
           35                  40                  45
   Thr Gly Ser Arg Ser Ala Ser Asn Met Ala Ile Val Asp Val Lys Met
       50                  55                  60
   Val Ser Gly Phe Ile Pro Leu Lys Pro Thr Val Lys Met Leu Glu Arg
   65                  70                  75                  80
   Ser Asn His Val Ser Arg Thr Glu Val Ser Ser Asn His Val Leu Ile
                   85                  90                  95
   Tyr Leu Asp Lys Val Ser Asn Gln Thr Leu Ser Leu Phe Phe Thr Val
               100                 105                 110
   Leu Gln Asp Val Pro Val Arg Asp Leu Lys Pro Ala Ile Val Lys Val
           115                 120                 125
   Tyr Asp Tyr Tyr Glu Thr Asp Glu Phe Ala Ile Ala Glu Tyr Asn Ala
       130                 135                 140
   Pro Cys Ser Lys Asp Leu Gly Asn Ala
   145                 150
<210> 7
   <211> 24
   <212> PRT
   <213> Artificial Sequence
<220>
   <223> HIV-Tat Minimum
<400> 7
   Arg Gly Asp Pro Thr Gly Gln Glu Glu Ser Lys Glu Lys Val Glu Lys
   1               5                   10                  15
   Glu Thr Val Val Asp Pro Val Thr
               20
<210> 8
   <211> 641
   <212> PRT
   <213> Artificial Sequence
<220>
   <223> HSPs Minimum
<400> 8
   Met Ala Lys Ala Ala Ala Ile Gly Ile Asp Leu Gly Thr Thr Tyr Ser
   1               5                   10                  15
   Cys Val Gly Val Phe Gln His Gly Lys Val Glu Ile Ile Ala Asn Asp
               20                  25                  30
   Gln Gly Asn Arg Thr Thr Pro Ser Tyr Val Ala Phe Thr Asp Thr Glu
           35                  40                  45
   Arg Leu Ile Gly Asp Ala Ala Lys Asn Gln Val Ala Leu Asn Pro Gln
       50                  55                  60
   Asn Thr Val Phe Asp Ala Lys Arg Leu Ile Gly Arg Lys Phe Gly Asp
   65                  70                  75                  80
   Pro Val Val Gln Ser Asp Met Lys His Trp Pro Phe Gln Val Ile Asn
                   85                  90                  95
   Asp Gly Asp Lys Pro Lys Val Gln Val Ser Tyr Lys Gly Glu Thr Lys
               100                 105                 110
   Ala Phe Tyr Pro Glu Glu Ile Ser Ser Met Val Leu Thr Lys Met Lys
           115                 120                 125
   Glu Ile Ala Glu Ala Tyr Leu Gly Tyr Pro Val Thr Asn Ala Val Ile
       130                 135                 140
   Thr Val Pro Ala Tyr Phe Asn Asp Ser Gln Arg Gln Ala Thr Lys Asp
   145                 150                 155                 160
   Ala Gly Val Ile Ala Gly Leu Asn Val Leu Arg Ile Ile Asn Glu Pro
                   165                 170                 175
   Thr Ala Ala Ala Ile Ala Tyr Gly Leu Asp Arg Thr Gly Lys Gly Glu
               180                 185                 190
   Arg Asn Val Leu Ile Phe Asp Leu Gly Gly Gly Thr Phe Asp Val Ser
           195                 200                 205
   Ile Leu Thr Ile Asp Asp Gly Ile Phe Glu Val Lys Ala Thr Ala Gly
       210                 215                 220
   Asp Thr His Leu Gly Gly Glu Asp Phe Asp Asn Arg Leu Val Asn His
   225                 230                 235                 240
   Phe Val Glu Glu Phe Lys Arg Lys His Lys Lys Asp Ile Ser Gln Asn
                   245                 250                 255
   Lys Arg Ala Val Arg Arg Leu Arg Thr Ala Cys Glu Arg Ala Lys Arg
               260                 265                 270
   Thr Leu Ser Ser Ser Thr Gln Ala Ser Leu Glu Ile Asp Ser Leu Phe
           275                 280                 285
   Glu Gly Ile Asp Phe Tyr Thr Ser Ile Thr Arg Ala Arg Phe Glu Glu
       290                 295                 300
   Leu Cys Ser Asp Leu Phe Arg Ser Thr Leu Glu Pro Val Glu Lys Ala
   305                 310                 315                 320
   Leu Arg Asp Ala Lys Leu Asp Lys Ala Gln Ile His Asp Leu Val Leu
                   325                 330                 335
   Val Gly Gly Ser Thr Arg Ile Pro Lys Val Gln Lys Leu Leu Gln Asp
               340                 345                 350
   Phe Phe Asn Gly Arg Asp Leu Asn Lys Ser Ile Asn Pro Asp Glu Ala
           355                 360                 365
   Val Ala Tyr Gly Ala Ala Val Gln Ala Ala Ile Leu Met Gly Asp Lys
       370                 375                 380
   Ser Glu Asn Val Gln Asp Leu Leu Leu Leu Asp Val Ala Pro Leu Ser
   385                 390                 395                 400
   Leu Gly Leu Glu Thr Ala Gly Gly Val Met Thr Ala Leu Ile Lys Arg
                   405                 410                 415
   Asn Ser Thr Ile Pro Thr Lys Gln Thr Gln Ile Phe Thr Thr Tyr Ser
               420                 425                 430
   Asp Asn Gln Pro Gly Val Leu Ile Gln Val Tyr Glu Gly Glu Arg Ala
           435                 440                 445
   Met Thr Lys Asp Asn Asn Leu Leu Gly Arg Phe Glu Leu Ser Gly Ile
       450                 455                 460
   Pro Pro Ala Pro Arg Gly Val Pro Gln Ile Glu Val Thr Phe Asp Ile
   465                 470                 475                 480
   Asp Ala Asn Gly Ile Leu Asn Val Thr Ala Thr Asp Lys Ser Thr Gly
                   485                 490                 495
   Lys Ala Asn Lys Ile Thr Ile Thr Asn Asp Lys Gly Arg Leu Ser Lys
               500                 505                 510
   Glu Glu Ile Glu Arg Met Val Gln Glu Ala Glu Lys Tyr Lys Ala Glu
           515                 520                 525
   Asp Glu Val Gln Arg Glu Arg Val Ser Ala Lys Asn Ala Leu Glu Ser
       530                 535                 540
   Tyr Ala Phe Asn Met Lys Ser Ala Val Glu Asp Glu Gly Leu Lys Gly
   545                 550                 555                 560
   Lys Ile Ser Glu Ala Asp Lys Lys Lys Val Leu Asp Lys Cys Gln Glu
                   565                 570                 575
   Val Ile Ser Trp Leu Asp Ala Asn Thr Leu Ala Glu Lys Asp Glu Phe
               580                 585                 590
   Glu His Lys Arg Lys Glu Leu Glu Gln Val Cys Asn Pro Ile Ile Ser
           595                 600                 605
   Gly Leu Tyr Gln Gly Ala Gly Gly Pro Gly Pro Gly Gly Phe Gly Ala
       610                 615                 620
   Gln Gly Pro Lys Gly Gly Ser Gly Ser Gly Pro Thr Ile Glu Glu Val
   625                 630                 635                 640
   Asp
<210> 9
   <211> 252
   <212> PRT
   <213> Pseudomonas aeruginosa
<400> 9
   Ala Glu Glu Ala Phe Asp Leu Trp Asn Glu Cys Ala Lys Ala Cys Val
   1               5                   10                  15
   Leu Asp Leu Lys Asp Gly Val Arg Ser Ser Arg Met Ser Val Asp Pro
               20                  25                  30
   Ala Ile Ala Asp Thr Asn Gly Gln Gly Val Leu His Tyr Ser Met Val
           35                  40                  45
   Leu Glu Gly Gly Asn Asp Ala Leu Lys Leu Ala Ile Asp Asn Ala Leu
       50                  55                  60
   Ser Ile Thr Ser Asp Gly Leu Thr Ile Arg Leu Glu Gly Gly Val Glu
   65                  70                  75                  80
   Pro Asn Lys Pro Val Arg Tyr Ser Tyr Thr Arg Gln Ala Arg Gly Ser
                   85                  90                  95
   Trp Ser Leu Asn Trp Leu Val Pro Ile Gly His Glu Lys Pro Ser Asn
               100                 105                 110
   Ile Lys Val Phe Ile His Glu Leu Asn Ala Gly Asn Gln Leu Ser His
           115                 120                 125
   Met Ser Pro Ile Tyr Thr Ile Glu Met Gly Asp Glu Leu Leu Ala Lys
   130 135 140
   Leu Ala Arg Asp Ala Thr Phe Phe Val Arg Ala His Glu Ser Asn Glu
   145 150 155 160
   Met Gln Pro Thr Leu Ala Ile Ser His Ala Gly Val Ser Val Val Met
   165 170 175
   Ala Gln Thr Gln Pro Arg Arg Glu Lys Arg Trp Ser Glu Trp Ala Ser
   180 185 190
   Gly Lys Val Leu Cys Leu Leu Asp Pro Leu Asp Gly Val Tyr Asn Tyr
   195 200 205
   Leu Ala Gln Gln Arg Cys Asn Leu Asp Asp Thr Trp Glu Gly Lys Ile
   210 215 220
   Tyr Arg Val Leu Ala Gly Asn Pro Ala Lys His Asp Leu Asp Ile Lys
   225 230 235 240
   Pro Thr Val Ile Ser His Arg Leu His Phe Pro Glu
   245 250
<210> 10
   <211> 613
   <212> PRT
   <213> Pseudomonas aeruginosa
<400> 10
   Ala Glu Glu Ala Phe Asp Leu Trp Asn Glu Cys Ala Lys Ala Cys Val
   1 5 10 15
   Leu Asp Leu Lys Asp Gly Val Arg Ser Ser Arg Met Ser Val Asp Pro
   20 25 30
   Ala Ile Ala Asp Thr Asn Gly Gln Gly Val Leu His Tyr Ser Met Val
   35 40 45
   Leu Glu Gly Gly Asn Asp Ala Leu Lys Leu Ala Ile Asp Asn Ala Leu
   50 55 60
   Ser Ile Thr Ser Asp Gly Leu Thr Ile Arg Leu Glu Gly Gly Val Glu
   65 70 75 80
   Pro Asn Lys Pro Val Arg Tyr Ser Tyr Thr Arg Gln Ala Arg Gly Ser
   85 90 95
   Trp Ser Leu Asn Trp Leu Val Pro Ile Gly His Glu Lys Pro Ser Asn
               100                 105                 110
   Ile Lys Val Phe Ile His Glu Leu Asn Ala Gly Asn Gln Leu Ser His
           115                 120                 125
   Met Ser Pro Ile Tyr Thr Ile Glu Met Gly Asp Glu Leu Leu Ala Lys
       130                 135                 140
   Leu Ala Arg Asp Ala Thr Phe Phe Val Arg Ala His Glu Ser Asn Glu
   145                 150                 155                 160
   Met Gln Pro Thr Leu Ala Ile Ser His Ala Gly Val Ser Val Val Met
                   165                 170                 175
   Ala Gln Thr Gln Pro Arg Arg Glu Lys Arg Trp Ser Glu Trp Ala Ser
               180                 185                 190
   Gly Lys Val Leu Cys Leu Leu Asp Pro Leu Asp Gly Val Tyr Asn Tyr
           195                 200                 205
   Leu Ala Gln Gln Arg Cys Asn Leu Asp Asp Thr Trp Glu Gly Lys Ile
       210                 215                 220
   Tyr Arg Val Leu Ala Gly Asn Pro Ala Lys His Asp Leu Asp Ile Lys
   225                 230                 235                 240
   Pro Thr Val Ile Ser His Arg Leu His Phe Pro Glu Gly Gly Ser Leu
                   245                 250                 255
   Ala Ala Leu Thr Ala His Gln Ala Cys His Leu Pro Leu Glu Thr Phe
               260                 265                 270
   Thr Arg His Arg Gln Pro Arg Gly Trp Glu Gln Leu Glu Gln Cys Gly
           275                 280                 285
   Tyr Pro Val Gln Arg Leu Val Ala Leu Tyr Leu Ala Ala Arg Leu Ser
       290                 295                 300
   Trp Asn Gln Val Asp Gln Val Ile Arg Asn Ala Leu Ala Ser Pro Gly
   305                 310                 315                 320
   Ser Gly Gly Asp Leu Gly Glu Ala Ile Arg Glu Gln Pro Glu Gln Ala
                   325                 330                 335
   Arg Leu Ala Leu Thr Leu Ala Ala Ala Glu Ser Glu Arg Phe Val Arg
               340                 345                 350
   Gln Gly Thr Gly Asn Asp Glu Ala Gly Ala Ala Asn Ala Asp Val Val
           355                 360                 365
   Ser Leu Thr Cys Pro Val Ala Ala Gly Glu Cys Ala Gly Pro Ala Asp
       370                 375                 380
   Ser Gly Asp Ala Leu Leu Glu Arg Asn Tyr Pro Thr Gly Ala Glu Phe
   385                 390                 395                 400
   Leu Gly Asp Gly Gly Asp Val Ser Phe Ser Thr Arg Gly Thr Gln Asn
                   405                 410                 415
   Trp Thr Val Glu Arg Leu Leu Gln Ala His Arg Gln Leu Glu Glu Arg
               420                 425                 430
   Gly Tyr Val Phe Val Gly Tyr His Gly Thr Phe Leu Glu Ala Ala Gln
           435                 440                 445
   Ser Ile Val Phe Gly Gly Val Arg Ala Arg Ser Gln Asp Leu Asp Ala
       450                 455                 460
   Ile Trp Arg Gly Phe Tyr Ile Ala Gly Asp Pro Ala Leu Ala Tyr Gly
   465                 470                 475                 480
   Tyr Ala Gln Asp Gln Glu Pro Asp Ala Arg Gly Arg Ile Arg Asn Gly
                   485                 490                 495
   Ala Leu Leu Arg Val Tyr Val Pro Arg Ser Ser Leu Pro Gly Phe Tyr
               500                 505                 510
   Arg Thr Ser Leu Thr Leu Ala Ala Pro Glu Ala Ala Gly Glu Val Glu
           515                 520                 525
   Arg Leu Ile Gly His Pro Leu Pro Leu Arg Leu Asp Ala Ile Thr Gly
       530                 535                 540
   Pro Glu Glu Glu Gly Gly Arg Leu Glu Thr Ile Leu Gly Trp Pro Leu
   545                 550                 555                 560
   Ala Glu Arg Thr Val Val Ile Pro Ser Ala Ile Pro Thr Asp Pro Arg
                   565                 570                 575
   Asn Val Gly Gly Asp Leu Asp Pro Ser Ser Ile Pro Asp Lys Glu Gln
               580                 585                 590
   Ala Ile Ser Ala Leu Pro Asp Tyr Ala Ser Gln Pro Gly Lys Pro Pro
           595                 600                 605
   Arg Glu Asp Leu Lys
   610
<210> 11
   <211> 323
   <212> PRT
   <213> Homo sapiens
<400> 11
   Tyr Ser Arg Glu Lys Asn Gln Pro Lys Pro Ser Pro Lys Arg Glu Ser
   1               5                   10                  15
   Gly Glu Glu Phe Arg Met Glu Lys Leu Asn Gln Leu Trp Glu Lys Ala
               20                  25                  30
   Gln Arg Leu His Leu Pro Pro Val Arg Leu Ala Glu Leu His Ala Asp
           35                  40                  45
   Leu Lys Ile Gln Glu Arg Asp Glu Leu Ala Trp Lys Lys Leu Lys Leu
       50                  55                  60
   Asp Gly Leu Asp Glu Asp Gly Glu Lys Glu Ala Arg Leu Ile Arg Asn
   65                  70                  75                  80
   Leu Asn Val Ile Leu Ala Lys Tyr Gly Leu Asp Gly Lys Lys Asp Ala
                   85                  90                  95
   Arg Gln Val Thr Ser Asn Ser Leu Ser Gly Thr Gln Glu Asp Gly Leu
               100                 105                 110
   Asp Asp Pro Arg Leu Glu Lys Leu Trp His Lys Ala Lys Thr Ser Gly
           115                 120                 125
   Lys Phe Ser Gly Glu Glu Leu Asp Lys Leu Trp Arg Glu Phe Leu His
       130                 135                 140
   His Lys Glu Lys Val His Glu Tyr Asn Val Leu Leu Glu Thr Leu Ser
   145                 150                 155                 160
   Arg Thr Glu Glu Ile His Glu Asn Val Ile Ser Pro Ser Asp Leu Ser
                   165                 170                 175
   Asp Ile Lys Gly Ser Val Leu His Ser Arg His Thr Glu Leu Lys Glu
               180                 185                 190
   Lys Leu Arg Ser Ile Asn Gln Gly Leu Asp Arg Leu Arg Arg Val Ser
           195                 200                 205
   His Gln Gly Tyr Ser Thr Glu Ala Glu Phe Glu Glu Pro Arg Val Ile
       210                 215                 220
   Asp Leu Trp Asp Leu Ala Gln Ser Ala Asn Leu Thr Asp Lys Glu Leu
   225                 230                 235                 240
   Glu Ala Phe Arg Glu Glu Leu Lys His Phe Glu Ala Lys Ile Glu Lys
                   245                 250                 255
   His Asn His Tyr Gln Lys Gln Leu Glu Ile Ala His Glu Lys Leu Arg
               260                 265                 270
   His Ala Glu Ser Val Gly Asp Gly Glu Arg Val Ser Arg Ser Arg Glu
           275                 280                 285
   Lys His Ala Leu Leu Glu Gly Arg Thr Lys Glu Leu Gly Tyr Thr Val
       290                 295                 300
   Lys Lys His Leu Gln Asp Leu Ser Gly Arg Ile Ser Arg Ala Arg His
   305                 310                 315                 320
   Asn Glu Leu
<210> 12
   <211> 357
   <212> PRT
   <213> Homo sapiens
<400> 12
   Met Ala Pro Arg Arg Val Arg Ser Phe Leu Arg Gly Leu Pro Ala Leu
   1               5                   10                  15
   Leu Leu Leu Leu Leu Phe Leu Gly Pro Trp Pro Ala Ala Ser His Gly
               20                  25                  30
   Gly Lys Tyr Ser Arg Glu Lys Asn Gln Pro Lys Pro Ser Pro Lys Arg
           35                  40                  45
   Glu Ser Gly Glu Glu Phe Arg Met Glu Lys Leu Asn Gln Leu Trp Glu
       50                  55                  60
   Lys Ala Gln Arg Leu His Leu Pro Pro Val Arg Leu Ala Glu Leu His
   65                  70                  75                  80
   Ala Asp Leu Lys Ile Gln Glu Arg Asp Glu Leu Ala Trp Lys Lys Leu
                   85                  90                  95
   Lys Leu Asp Gly Leu Asp Glu Asp Gly Glu Lys Glu Ala Arg Leu Ile
               100                 105                 110
   Arg Asn Leu Asn Val Ile Leu Ala Lys Tyr Gly Leu Asp Gly Lys Lys
           115                 120                 125
   Asp Ala Arg Gln Val Thr Ser Asn Ser Leu Ser Gly Thr Gln Glu Asp
       130                 135                 140
   Gly Leu Asp Asp Pro Arg Leu Glu Lys Leu Trp His Lys Ala Lys Thr
   145                 150                 155                 160
   Ser Gly Lys Phe Ser Gly Glu Glu Leu Asp Lys Leu Trp Arg Glu Phe
                   165                 170                 175
   Leu His His Lys Glu Lys Val His Glu Tyr Asn Val Leu Leu Glu Thr
               180                 185                 190
   Leu Ser Arg Thr Glu Glu Ile His Glu Asn Val Ile Ser Pro Ser Asp
           195                 200                 205
   Leu Ser Asp Ile Lys Gly Ser Val Leu His Ser Arg His Thr Glu Leu
       210                 215                 220
   Lys Glu Lys Leu Arg Ser Ile Asn Gln Gly Leu Asp Arg Leu Arg Arg
   225                 230                 235                 240
   Val Ser His Gln Gly Tyr Ser Thr Glu Ala Glu Phe Glu Glu Pro Arg
                   245                 250                 255
   Val Ile Asp Leu Trp Asp Leu Ala Gln Ser Ala Asn Leu Thr Asp Lys
               260                 265                 270
   Glu Leu Glu Ala Phe Arg Glu Glu Leu Lys His Phe Glu Ala Lys Ile
           275                 280                 285
   Glu Lys His Asn His Tyr Gln Lys Gln Leu Glu Ile Ala His Glu Lys
       290                 295                 300
   Leu Arg His Ala Glu Ser Val Gly Asp Gly Glu Arg Val Ser Arg Ser
   305                 310                 315                 320
   Arg Glu Lys His Ala Leu Leu Glu Gly Arg Thr Lys Glu Leu Gly Tyr
                   325                 330                 335
   Thr Val Lys Lys His Leu Gln Asp Leu Ser Gly Arg Ile Ser Arg Ala
   340 345 350
   Arg His Asn Glu Leu
   355
<210> 13
   <211> 101
   <212> PRT
   <213> Human immunodeficiency virus
<400> 13
   Met Glu Pro Val Asp Pro Arg Leu Glu Pro Trp Lys His Pro Gly Ser
   1 5 10 15
   Gln Pro Lys Thr Pro Cys Thr Lys Cys Tyr Cys Lys Lys Cys Cys Leu
   20 25 30
   His Cys Gln Val Cys Phe Met Thr Lys Gly Leu Gly Ile Ser Tyr Gly
   35 40 45
   Arg Lys Lys Arg Arg Gln Arg Arg Arg Ala Pro Gln Asp Asn Lys Asn
   50 55 60
   His Gln Val Ser Leu Ser Lys Gln Pro Thr Ser Arg Ala Arg Gly Asp
   65 70 75 80
   Pro Thr Gly Gln Glu Glu Ser Lys Glu Lys Val Glu Lys Glu Thr Val
   85 90 95
   Val Asp Pro Val Thr
   100
<210> 14
   <211> 4
   <212> PRT
   <213> Artificial Sequence
<220>
   <223> ER retention sequence
<400> 14
   Lys Asp Glu Leu
   1
<210> 15
   <211> 6
   <212> PRT
   <213> Artificial Sequence
<220>
   <223> Linker of CD28-PEt
<220>
   <221> misc_feature
   <222> (2)..(2)
   <223> Xaa can be any naturally occurring amino acid
<220>
   <221> misc_feature
   <222> (4)..(4)
   <223> Xaa can be any naturally occurring amino acid
<400> 15
   Arg Xaa Arg Xaa Lys Arg
   1 5
<210> 16
   <211> 12
   <212> PRT
   <213> Artificial Sequence
<220>
   <223> ER retention sequence
<400> 16
   Lys Lys Asp Leu Arg Asp Glu Leu Lys Asp Glu Leu
   1 5 10
<210> 17
   <211> 13
   <212> PRT
   <213> Artificial Sequence
<220>
   <223> ER retention sequence
<400> 17
   Lys Lys Asp Glu Leu Arg Asp Glu Leu Lys Asp Glu Leu
   1 5 10
<210> 18
   <211> 13
   <212> PRT
   <213> Artificial Sequence
<220>
   <223> ER retention sequence
<400> 18
   Lys Lys Asp Glu Leu Arg Val Glu Leu Lys Asp Glu Leu
   1 5 10
<210> 19
   <211> 12
   <212> PRT
   <213> Artificial Sequence
<220>
   <223> ER retention sequence
<400> 19
   Lys Asp Glu Leu Lys Asp Glu Leu Lys Asp Glu Leu
   1               5                   10
<210> 20
   <211> 46
   <212> PRT
   <213> Pseudomonas aeruginosa
<400> 20
   Pro Leu Glu Thr Phe Thr Arg His Arg Gln Pro Arg Gly Trp Glu Gln
   1               5                   10                  15
   Leu Glu Gln Cys Gly Tyr Pro Val Gln Arg Leu Val Ala Leu Tyr Leu
               20                  25                  30
   Ala Ala Arg Leu Ser Trp Asn Gln Val Asp Gln Val Ile Arg
           35                  40                  45
<210> 21
   <211> 98
   <212> PRT
   <213> Human papillomavirus type 16
<400> 21
   Met His Gly Asp Thr Pro Thr Leu His Glu Tyr Met Leu Asp Leu Gln
   1               5                   10                  15
   Pro Glu Thr Thr Asp Leu Tyr Cys Tyr Glu Gln Leu Asn Asp Ser Ser
               20                  25                  30
   Glu Glu Glu Asp Glu Ile Asp Gly Pro Ala Gly Gln Ala Glu Pro Asp
           35                  40                  45
   Arg Ala His Tyr Asn Ile Val Thr Phe Cys Cys Lys Cys Asp Ser Thr
       50                  55                  60
   Leu Arg Leu Cys Val Gln Ser Thr His Val Asp Ile Arg Thr Leu Glu
   65                  70                  75                  80
   Asp Leu Leu Met Gly Thr Leu Gly Ile Val Cys Pro Ile Cys Ser Gln
                   85                  90                  95
   Lys Pro
<210> 22
   <211> 105
   <212> PRT
   <213> Human papillomavirus type 18
<400> 22
   Met His Gly Pro Lys Ala Thr Leu Gln Asp Ile Val Leu His Leu Glu
   1               5                   10                  15
   Pro Gln Asn Glu Ile Pro Val Asp Leu Leu Cys His Glu Gln Leu Ser
               20                  25                  30
   Asp Ser Glu Glu Glu Asn Asp Glu Ile Asp Gly Val Asn His Gln His
           35                  40                  45
   Leu Pro Ala Arg Arg Ala Glu Pro Gln Arg His Thr Met Leu Cys Met
       50                  55                  60
   Cys Cys Lys Cys Glu Ala Arg Ile Lys Leu Val Val Glu Ser Ser Ala
   65                  70                  75                  80
   Asp Asp Leu Arg Ala Phe Gln Gln Leu Phe Leu Asn Thr Leu Ser Phe
                   85                  90                  95
   Val Cys Pro Trp Cys Ala Ser Gln Gln
               100                 105
<210> 23
   <211> 190
   <212> PRT
   <213> Hepatitis C virus
<400> 23
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Asn Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Val Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Thr Pro Ala Ser
               180                 185                 190
<210> 24
   <211> 154
   <212> PRT
   <213> Hepatitis B virus
<400> 24
   Met Ala Ala Arg Met Cys Cys Gln Leu Asp Pro Ala Arg Asp Val Leu
   1               5                   10                  15
   Cys Leu Arg Pro Val Gly Ala Glu Ser Arg Gly Arg Pro Leu Pro Gly
               20                  25                  30
   Pro Leu Gly Ala Leu Pro Pro Ser Ser Ala Ser Ala Val Pro Ala Asp
           35                  40                  45
   His Gly Ser His Leu Ser Leu Arg Gly Leu Pro Val Cys Ser Phe Ser
       50                  55                  60
   Ser Ala Gly Pro Cys Ala Leu Arg Phe Thr Ser Ala Arg Arg Met Glu
   65                  70                  75                  80
   Thr Thr Val Asn Ala Pro Trp Ser Leu Pro Thr Val Leu His Lys Arg
                   85                  90                  95
   Thr Ile Gly Leu Ser Gly Arg Ser Met Thr Trp Ile Glu Glu Tyr Ile
               100                 105                 110
   Lys Asp Cys Val Phe Lys Asp Trp Glu Glu Leu Gly Glu Glu Ile Arg
           115                 120                 125
   Leu Lys Val Phe Val Leu Gly Gly Cys Arg His Lys Leu Val Cys Ser
       130                 135                 140
   Pro Ala Pro Cys Asn Phe Phe Thr Ser Ala
   145                 150
<210> 25
   <211> 192
   <212> PRT
   <213> Porcine circovirus
<400> 25
   Asn Gly Ile Phe Asn Thr Arg Leu Ser Arg Thr Phe Gly Tyr Thr Ile
   1               5                   10                  15
   Lys Arg Thr Thr Val Lys Thr Pro Ser Trp Ala Val Asp Met Met Arg
               20                  25                  30
   Phe Asn Ile Asn Asp Phe Leu Pro Pro Gly Gly Gly Ser Asn Pro Arg
           35                  40                  45
   Ser Val Pro Phe Glu Tyr Tyr Ser Ile Ser Lys Val Lys Val Glu Phe
       50                  55                  60
   Trp Pro Cys Ser Pro Ile Thr Gln Gly Asp Ser Gly Val Gly Ser Ser
   65                  70                  75                  80
   Ala Val Ile Leu Asp Asp Asn Phe Val Thr Lys Ala Thr Ala Leu Thr
                   85                  90                  95
   Tyr Asp Pro Tyr Val Asn Tyr Ser Ser Arg His Thr Ile Thr Gln Pro
               100                 105                 110
   Phe Ser Tyr His Ser Arg Tyr Phe Thr Pro Lys Pro Val Leu Asp Ser
           115                 120                 125
   Thr Ile Asp Tyr Phe Gln Pro Asn Asn Lys Arg Asn Gln Leu Trp Leu
       130                 135                 140
   Arg Leu Gln Thr Ala Gly Asn Val Asp His Val Gly Leu Gly Thr Ala
   145                 150                 155                 160
   Phe Glu Asn Ser Ile Tyr Asp Gln Glu Tyr Asn Ile Arg Val Thr Met
                   165                 170                 175
   Tyr Val Gln Phe Arg Glu Phe Asn Leu Lys Asp Pro Pro Leu Asn Pro
               180                 185                 190
<210> 26
   <211> 220
   <212> PRT
   <213> Porcine reproductive and respiratory syndrome virus
<400> 26
   Arg His His Phe Thr Pro Ser Glu Arg Gln Leu Cys Leu Ser Ser Ile
   1               5                   10                  15
   Gln Thr Ala Phe Asn Gln Gly Ala Gly Thr Cys Ile Leu Ser Asp Ser
               20                  25                  30
   Gly Arg Ile Ser Tyr Thr Val Glu Phe Ser Leu Pro Thr His His Thr
           35                  40                  45
   Val Arg Leu Ile Arg Val Thr Ala Pro Pro Ser Ala Leu Asp Gln Val
       50                  55                  60
   Ile Arg Asn Ala Leu Ala Ser Pro Gly Ser Gly Gly Asp Leu Gly Glu
   65                  70                  75                  80
   Ala Ile Arg Glu Gln Pro Glu Gln Ala Arg Leu Ala Leu Thr Leu Ala
                   85                  90                  95
   Ala Ala Glu Ser Glu Arg Phe Val Arg Gln Gly Thr Gly Asn Asp Glu
               100                 105                 110
   Ala Gly Ala Ala Asn Ala Asp Val Val Ser Leu Thr Cys Pro Val Ala
           115                 120                 125
   Ala Gly Glu Cys Ala Gly Pro Ala Asp Ser Gly Asp Ala Leu Leu Glu
       130                 135                 140
   Arg Asn Tyr Pro Thr Gly Ala Glu Phe Leu Gly Asp Gly Gly Asp Val
   145                 150                 155                 160
   Arg His His Phe Thr Pro Ser Glu Arg Gln Leu Cys Leu Ser Ser Ile
                   165                 170                 175
   Gln Thr Ala Phe Asn Gln Gly Ala Gly Thr Cys Ile Leu Ser Asp Ser
               180                 185                 190
   Gly Arg Ile Ser Tyr Thr Val Glu Phe Ser Leu Pro Thr His His Thr
           195                 200                 205
   Val Arg Leu Ile Arg Val Thr Ala Pro Pro Ser Ala
       210                 215                 220
<210> 27
   <211> 165
   <212> PRT
   <213> Porcine reproductive and respiratory syndrome virus
<400> 27
   Asn Asn Lys Glu Cys Thr Val Ala Gln Ala Leu Gly Asn Gly Asp Lys
   1               5                   10                  15
   Phe Arg Ala Thr Asp Lys Arg Val Val Asp Ser Leu Arg Ala Ile Cys
               20                  25                  30
   Ala Asp Leu Glu Gly Ser Ser Ser Pro Leu Pro Lys Val Ala His Asn
           35                  40                  45
   Leu Gly Phe Tyr Phe Ser Pro Asp Leu Thr Gln Phe Ala Lys Leu Pro
       50                  55                  60
   Ile Glu Leu Asp Pro His Trp Pro Val Val Ser Thr Gln Asn Asn Glu
   65                  70                  75                  80
   Lys Trp Pro Asp Arg Leu Val Ala Ser Leu Arg Pro Leu Asp Lys Tyr
                   85                  90                  95
   Ser Arg Ala Cys Ile Gly Ala Gly Tyr Met Val Gly Pro Ser Val Phe
               100                 105                 110
   Leu Gly Thr Pro Gly Val Val Ser Tyr Tyr Leu Thr Lys Phe Val Lys
           115                 120                 125
   Gly Glu Ala Gln Val Leu Pro Glu Thr Val Phe Ser Thr Gly Arg Ile
       130                 135                 140
   Glu Val Asp Cys Arg Glu Tyr Leu Asp Asp Arg Glu Arg Glu Val Ala
   145                 150                 155                 160
   Ala Ser Leu Pro His
                   165
<210> 28
   <211> 58
   <212> PRT
   <213> Porcine reproductive and respiratory syndrome virus
<400> 28
   Gly Ser Ser Leu Asp Asp Phe Cys Tyr Asp Ser Thr Ala Pro Gln Lys
   1 5 10 15
   Val Leu Leu Ala Phe Ser Ile Thr Tyr Ala Ser Asn Asp Ser Ser Ser
   20 25 30
   His Leu Gln Leu Ile Tyr Asn Leu Thr Leu Cys Glu Leu Asn Gly Thr
   35 40 45
   Asp Trp Leu Ala Asn Lys Phe Asp Trp Ala
   50 55
<210> 29
   <211> 62
   <212> PRT
   <213> Porcine reproductive and respiratory syndrome virus
<400> 29
   Met Gly Ser Leu Asp Asp Phe Cys Asn Asp Ser Thr Ala Ala Gln Lys
   1 5 10 15
   Leu Val Leu Ala Phe Ser Ile Thr Tyr Thr Pro Ile Phe Val Ala Gly
   20 25 30
   Gly Ser Ser Ser Thr Tyr Gln Tyr Ile Tyr Asn Leu Thr Ile Cys Glu
   35 40 45
   Leu Asn Gly Thr Asp Trp Leu Ser Asn His Phe Asp Trp Ala
   50 55 60
<210> 30
   <211> 68
   <212> PRT
   <213> Artificial Sequence
<220>
   <223> CD28-PEt
<220>
   <221> misc_feature
   <222> (8)..(8)
   <223> Xaa can be any naturally occurring amino acid
<220>
   <221> misc_feature
   <222> (10)..(11)
   <223> Xaa can be any naturally occurring amino acid
<220>
   <221> misc_feature
   <222> (17)..(17)
   <223> Xaa can be any naturally occurring amino acid
<220>
   <221> misc_feature
   <222> (30)..(30)
   <223> Xaa can be any naturally occurring amino acid
<220>
   <221> misc_feature
   <222> (32)..(32)
   <223> Xaa can be any naturally occurring amino acid
<400> 30
   Thr Asp Ile Tyr Phe Cys Lys Xaa Glu Xaa Xaa Tyr Pro Pro Pro Tyr
   1               5                   10                  15
   Xaa Asp Asn Glu Lys Ser Asn Gly Thr Ile Ile His Arg Xaa Arg Xaa
               20                  25                  30
   Lys Arg Gly Trp Glu Gln Leu Glu Gln Cys Gly Tyr Pro Val Gln Arg
           35                  40                  45
   Leu Val Ala Leu Tyr Leu Ala Ala Arg Leu Ser Trp Asn Gln Val Asp
       50                  55                  60
   Gln Val Ile Arg
   65
<210> 31
   <211> 28
   <212> PRT
   <213> Artificial Sequence
<220>
   <223> CD28 consensus sequence
<220>
   <221> misc_feature
   <222> (8)..(8)
   <223> Xaa can be any naturally occurring amino acid
<220>
   <221> misc_feature
   <222> (10)..(11)
   <223> Xaa can be any naturally occurring amino acid
<220>
   <221> misc_feature
   <222> (17)..(17)
   <223> Xaa can be any naturally occurring amino acid
<400> 31
   Thr Asp Ile Tyr Phe Cys Lys Xaa Glu Xaa Xaa Tyr Pro Pro Pro Tyr
   1               5                   10                  15
   Xaa Asp Asn Glu Lys Ser Asn Gly Thr Ile Ile His
                       20                  25
<210> 32
   <211> 10
   <212> PRT
   <213> Artificial Sequence
<220>
   <223> CD28 critical region
<220>
   <221> misc_feature
   <222> (2)..(2)
   <223> Xaa can be any naturally occurring amino acid
<220>
   <221> misc_feature
   <222> (4)..(5)
   <223> Xaa can be any naturally occurring amino acid
<400> 32
   Lys Xaa Glu Xaa Xaa Tyr Pro Pro Pro Tyr
   1               5                   10

## Description

### FIELD OF THE INVENTION

The present invention relates generally to fusion proteins, and more specifically to fusion proteins for enhancing T cell-mediated immune response.

### BACKGROUND OF THE INVENTION

Molecular biology has enabled the production of subunit vaccines, in which the immunogen is a fragment or subunit of a parent protein or complex. The development of a stable vaccine that could elicit T cell sensitizing responses, and be flexible enough to incorporate sequences from many strains of an infectious agent would be desirable.

US 2009/214570 A1 disclose methods and compositions for immunizing against *Chlamydia* infection. Takemoto el al. disclose Enhanced generation of cytotoxic T lymphocytes by heat shock protein 70 fusion proteins harboring both CD8(+) T cell and CD4(+) T cell epitopes. Mol Pharm. 2010 VOL, NO. 5, 1715-1723.

### SUMMARY OF THE INVENTION

In one aspect, the invention relates to a fusion protein comprising:
(a) an antigen-presenting cell (APC)-binding domain or a CD91 receptor-binding domain, located at the N-terminus of the fusion protein, wherein the APC-binding domain or CD91 receptor-binding domain is selected from the group consisting of receptor-associated protein-1 (RAP1) domain III, alpha-2-macroglobulin receptor-associated protein (A2M), HIV-Tat, heat shock 70kDa protein, and Pseudomonas exotoxin A binding domain la;
(b) a protein transduction domain, located at the C-terminus of the APC-binding domain or the CD91 receptor-binding domain, the protein transduction domain is a fusion polypeptide, comprising a T cell sensitizing signal-transducing peptide, a linker, and a translocation peptide, wherein:
   (1) the T cell sensitizing signal-transducing peptide is located at the N-terminus of the fusion polypeptide;
   (2) the linker comprises SEQ ID NO: 15, linking the T cell sensitizing signal-transducing peptide and the translocation peptide; and
   (3) the translocation peptide has 34-112 amino acid residues in length and comprises an amino acid sequence consisting of SEQ ID NO: 3, 20 or 4; and
(c) an antigen of a pathogen, located at the C-terminus of the protein transduction domain; wherein:
   the T cell-sensitizing signal-transducing peptide has 28-53 amino acid residues in length and comprises the amino acid sequence of SEQ ID NO: 31, in which Xaa⁸ is I or L; Xaa¹⁰ is V, F or A, Xaa¹¹ is M or L, Xaa¹⁷ is L or I.

In one embodiment of the invention, the APC-binding domain or the CD91 receptor-binding domain is a polypeptide comprising an amino acid sequence that is at least 90% identical to the sequence selected from the group consisting of SEQ ID NOs: 5, 9, 6, 7, and 8. Alternatively, the APC-binding domain is selected from the group consisting of receptor-associated protein-1 (RAP1) domain III, alpha-2-macroglobulin receptor-associated protein (A2M), HIV-Tat, and heat shock proteins (HSPs), and Pseudomonas exotoxin A (PE) binding domain Ia.

In another embodiment of the invention, the fusion protein is free of the amino acid sequence of Pseudomonas exotoxin A (PE) binding domain Ia.

In another embodiment of the invention, the fusion protein further comprises an endoplasmic reticulum retention sequence located at the C-terminus of the fusion protein.

In another embodiment of the invention, the endoplasmic reticulum retention sequence comprises the amino acid sequence of Lys-Asp-Glu-Leu (SEQ ID NO: 14). The ER retention sequence may comprise a sequence selected from the group consisting of SEQ ID NOs: 14, 16-19. Alternatively, the ER retention sequence may consist of a sequence selected from the group consisting of SEQ ID NOs: 16-19.

In another embodiment of the invention, the fusion protein is free of an endoplasmic reticulum retention sequence at C-terminus thereof if the antigen contains 10 or more epitopes.

In another embodiment of the invention, the protein transduction domain comprises the sequence of SEQ ID NO: 30.

In another embodiment of the invention, the APC-binding domain comprises an amino acid sequence that is at least 95% identical to the sequence selected from the group consisting of SEQ ID NOs: 5, 9, 6, 7, and 8.

In another embodiment of the invention, the APC-binding domain or the CD91 receptor-binding domain is a polypeptide comprising an amino acid sequence selected from the group consisting of SEQ ID NOs: 5, 9, 6, 7, and 8.

In another embodiment of the invention, the T cell sensitizing signal-transducing peptide comprises an amino acid sequence selected from the group consisting of SEQ ID NO: 1 and 2.

In another embodiment of the invention, the translocation peptide comprises the amino acid sequence of SEQ ID NO: 3.

In another embodiment of the invention, the translocation peptide has 34-61 amino acid residues in length.

In another embodiment of the invention, the protein transduction domain of the fusion protein of the invention possesses the following features: (i) the T cell-sensitizing signal-transducing peptide comprises the amino acid sequence of SEQ ID NO: 1 or 2; and (ii) the translocation peptide comprises the amino acid sequence that is at least 95% identical to SEQ ID NO: 3.

The T cell sensitizing signal-transducing peptide exhibits a characteristic of eliciting an antibody that recognizes and binds to the amino acid sequence of K¹(X)²E³(X)⁴(X)⁵Y⁶P⁷P⁸P⁹Y¹⁰ (SEQ ID NO: 32) of CD28 receptor on T cells, wherein (X)² is I or L; (X)⁴ is V, F or A, and (X)⁵ is M or L.

In another aspect, the invention relates to a fusion protein consisting of:
(a) an antigen-presenting cell (APC)-binding domain or a CD91 receptor-binding domain, located at the N-terminus of the fusion protein, wherein the APC-binding domain or CD91 receptor-binding domain is selected from the group consisting of receptor-associated protein-1 (RAP1) domain III, alpha-2-macroglobulin receptor-associated protein (A2M), HIV-Tat, heat shock 70kDa protein, and Pseudomonas exotoxin A binding domain la;
(b) a protein transduction domain, located at the C-terminus of the APC-binding domain or the CD91 receptor-binding domain, the protein transduction domain is a fusion polypeptide, comprising a T cell sensitizing signal-transducing peptide, a linker, and a translocation peptide, wherein:
   (1) the T cell sensitizing signal-transducing peptide is located at the N-terminus of the fusion polypeptide;
   (2) the linker comprises SEQ ID NO: 15, linking the T cell sensitizing signal-transducing peptide and the translocation peptide; and
   (3) the translocation peptide has 34-112 amino acid residues in length and comprises an amino acid sequence that is at least 90% identical to SEQ ID NO: 3, 20 or 4; and
(c) an antigen of a pathogen, located at the C-terminus of the protein transduction domain;
wherein:
the T cell-sensitizing signal-transducing peptide has 28-53 amino acid residues in length and comprises the amino acid sequence of SEQ ID NO: 31, in which Xaa⁸ is I or L; Xaa¹⁰ is V, F or A, Xaa¹¹ is M or L, Xaa¹⁷ is L or I.

The antigen-presenting cell (APC) may be selected from the group consisting of dendritic cells, macrophages, B-cells and monocytes.

In one embodiment of the invention, the cell membrane of the APC comprises a CD91 receptor.

In another aspect, the invention relates to a vaccine composition comprising: (a) a therapeutically effective amount of a fusion protein of the invention; and (b) an adjuvant.

The adjuvant is either an antigen delivery agent or an immune potentiator. In one embodiment of the invention, the vaccine composition comprises an antigen delivery agent and is free of an immune potentiator.

The invention also relates to a fusion protein or a vaccine composition as aforementioned, for use in inducing enhanced pathogen antigen-specific T cell responses, or for use in killing a disease cell that presents an antigen via class I MHC molecules on the cell membrane of the disease cell, or for use in preventing, treating infection caused by a pathogen, and/or minimizing symptoms caused by the infection. In one embodiment of the invention, the disease cell is a cancer cell.

The pathogen may be at least one selected from the group consisting of Human Papillomavirus (HPV), Porcine Reproductive and Respiratory Syndrome Virus (PRRSV), Human Immuno-deficient Virus (HIV-1), flu virus, Dengue virus, Hepatitis C virus (HCV), Hepatitis B virus (HBV) and Porcine Circovirus 2 (PCV2).

In one embodiment of the invention, the fusion protein as aforementioned is for use in enhancing an antigen-specific cytotoxic T cell response in a subject in need thereof. The fusion protein may also be for use in enhancing an antigen-specific CD4+ T cell response, or for use as an immunogenic enhancer for inducing an enhanced antigen-specific antibody titer response, in a subject in need thereof.

These and other aspects will become apparent from the following description of the preferred embodiment taken in conjunction with the following drawings.

The accompanying drawings illustrate one or more embodiments of the invention and, together with the written description, serve to explain the principles of the invention. Wherever possible, the same reference numbers are used throughout the drawings to refer to the same or like elements of an embodiment.

### BRIEF DESCRIPTION OF THE DRAWINGS

FIG. 1 is a vector map.
FIG. 2 is a schematic drawing illustrating one embodiment of the invention.
FIGs. 3A-B are a flow chart for preparation of fusion proteins and a photograph showing the result of SDS-PAGE analyses of fusion proteins, respectively.
FIG. 4 is a schematic drawing illustrating the mechanisms of actions of T cell-sensitizing fusion proteins.
FIG. 5 shows sequence alignments of CD28 from various species: human (SEQ ID NO: 33), rat (SEQ ID NO: 34), mouse (SEQ ID NO: 35), rabbit (SEQ ID NO: 36), pig (SEQ ID NO: 37), bovine (SEQ ID NO: 38), sheep (SEQ ID NO: 39), dog (SEQ ID NO: 40), horse (SEQ ID NO: 41), turkey (SEQ ID NO: 42), and the consensus sequence SEQ ID NO: 43.
FIG. 6A shows immunization schedules.
FIGs. 6B-C show tumor size curves and survival rate in the animal groups vaccinated with various fusion proteins or placebo, respectively.
FIG. 7 is a schematic drawing showing a RAP1-containing vector used for generating a plasmid containing a DNA insert from a pathogen.
FIG. 8 are schematic drawings illustrating constructions of fusion proteins containing antigens of various pathogens.
FIGs. 9A-F are photographs showing the results of SDS-PAGE analyses of various fusion proteins.
FIGs. 10 A-B show animal groups, vaccines and dosage used for immunizing the animals, and immunization schedules.
FIGs. 11A-D are tables showing the results of *ex vivo* antigen-specific immune response analyses of CD3+/CD4+ splenocytes and CD3+/CD8+ splenocytes from the animal groups of FIG. 10A vaccinated with placebo or a fusion protein containing E7₁₆, E7₁₈, HCVcore, or HBx antigen.
FIGs. 12A-J show IFNγ+ cell counts in *ex vivo* antigen-specific immune response analyses of CD3+/CD8+ splenocytes and CD3+/CD4+ splenocytes from the animal groups of FIG. 10A vaccinated with placebo or a fusion protein containing a PCV2 (12A-B) or PRRSV antigen (12C-J). Fusion proteins containing PCV2 or PRRSV antigen are shown in FIG. 10A.

### DETAILED DESCRIPTION OF THE INVENTION

The present invention is more particularly described in the following examples that are intended as illustrative only.

### DEFINITIONS

The term "an antigen-presenting cell (APC) or accessory cell" refers to a cell that displays foreign antigens complexed with major histocompatibility complexes (MHC's) on their surfaces. T-cells may recognize these complexes using their T-cell receptors (TCRs). These cells process antigens and present them to T-cells. Main types of professional antigen-presenting cell: dendritic cells (DCs), macrophages, monocytes, and certain B-cells.

The term "an antigen-presenting cell (APC)-binding domain" refers to a domain that can bind to an antigen-presenting cell (APC). The APC-binding domain may be a polypeptide comprising an amino acid sequence that is at least 90% identical to the sequence selected from the group consisting of SEQ ID NOs: 5, 6, 7, 8, and 9. An APC-binding domain is a ligand that recognizes and binds to a receptor on

Cluster of differentiation 91 (CD91) is a protein that forms a receptor in the membrane of cells and is involved in receptor-mediated endocytosis.

The term "a protein transduction domain" refers to a polypeptide or a fusion polypeptide having a function to sensitize T-cells and thus enhance antigen-specific T cell responses, and/or to guide or direct an antigen toward (i.e., to target to) class I major histocompatibility complex (MHC-I) pathway (i.e., a cytotoxic T cell pathway) of antigen presentation.

The term "to sensitize T cells" generally means that CD8+ and CD4+ T cells are sensitized and as a result, CD8+ (CTL) and CD4+ T cell responses to an antigen challenge are enhanced. An antigen-specific cell mediated immune response is measured by quantifying the production of antigen-specific induced γ-interferon in response to an antigen. For example, without a sensitization signal (i.e., without the protein transduction domain), an antigen alone may induce weak or no cell mediated immune response at all, i.e., weak or no production of antigen-specific γ-interferon from CD8+ and CD4+ T cells, while in the presence of a sensitization signal (the protein transduction domain), the antigen may induce an enhanced cell mediated immune response. Thus, the function of a sensitization signal (the protein transduction domain) is to sensitize CD4+ and CD8+ T cells in a host so that when the host is later challenged by an antigen, the antigen can induce an enhanced antigen-specific cell mediated immune response due to prior CD4+ and CD8+ T cell sensitization.

A protein transduction domain may be a fusion polypeptide, comprising a T cell sensitizing signal-transducing peptide, a linker, and a translocation peptide, wherein:
(1) the T cell sensitizing signal-transducing peptide is located at the N-terminus of the fusion polypeptide;
(2) the linker comprises SEQ ID NO: 15, linking the T cell sensitizing signal-transducing peptide and the translocation peptide; and
(3) the translocation peptide has 34-112 amino acid residues in length and comprises the amino acid sequence consisting of SEQ ID NO: 3, 20 or 4.

A protein transduction domain may be "a fusion polypeptide", in which the fusion polypeptide comprises a T cell sensitizing signal-transducing peptide, a linker, and a translocation peptide. For example, the fusion polypeptide may be the polypeptide "CD28convPEₜ".

The term "CD28conv" refers to a CD28 conserved region, which is a "T cell sensitizing signal-transducing peptide". It's an epitope for inducing CD28 agonist antibody.

The term "PEₜ" or "PEₜ Core" refers to a PE translocation domain core with 34 amino acid residues in length.

A linker is present between the "CD28conv" and the "PEₜ". The orientation or arrangement of the fusion polypeptide "CD28convPEₜ" is important in that "CD28conv" (or the T cell sensitizing signal-transducing peptide) must be at the upstream to the PEₜ (or the translocation peptide), i.e., PEₜ must be at the C-terminus of the "CD28conv" to obtain enhanced T-cell responses. The "CD28convPEₜ" can raise much higher IgG titer (called CD28-specific agonist antibody) specific to CD28conv than the reversed orientation fusion peptide PEₜCD28conv. The CD28-specific agonist antibody can sensitize both CD4+ and CD8+ T cells. The correct orientation fusion polypeptide CD28convPEₜ contains a linker (RXRXKR) between CD28conv and PEₜ domains. The linker contains an antigen presenting cell (APC)-specific protease (cathepsin L) cutting site Lys-Arg (KR). Therefore, the fusion protein RAP1-CD28convPEₜ-Antigen-K3 can be digested into the two fragments: RAP1-CD28conv and PEₜ-Antigen-K3. The RAP1-CD28conv fragment can be further digested in the lysosome and the epitope of CD28conv is then presented to the APC cell surface via MHC II pathway, which in turn elicits a humoral immune response producing CD28 agonist antibody. Thus, CD28 agonist antibody is produced by B cells. This CD28 agonist antibody can bind to CD28 on the T cell surface and pre-activate the T cells (CD4+ and CD8+ T cells).

A "T cell-sensitizing signal-transducing peptide" has 28-53 amino acid residues in length and comprises an amino acid sequence that is at least 90% identical to SEQ ID NO: 31, in which Xaa⁸ is I or L; Xaa¹⁰ is V, F or A, Xaa¹¹ is M or L, Xaa¹⁷ is L or I.

The T cell-sensitizing signal-transducing peptide comprises the critical region K¹(I/L)²E³(V/F/A)⁴(M/L)⁵Y⁶P⁷P⁸P⁹Y¹⁰ (SEQ ID NO: 32), wherein (X)² is I or L; (X)⁴ is V, F or A, (X)⁵ is M or L.

A T cell sensitizing signal-transducing peptide (TDIYFCKIEFMYPPPYLDNEKSNGTIIH; SEQ ID NO: 31, wherein X⁸ is I, X¹⁰ is F, X¹¹ is M) specific for mice was used in the following examples.

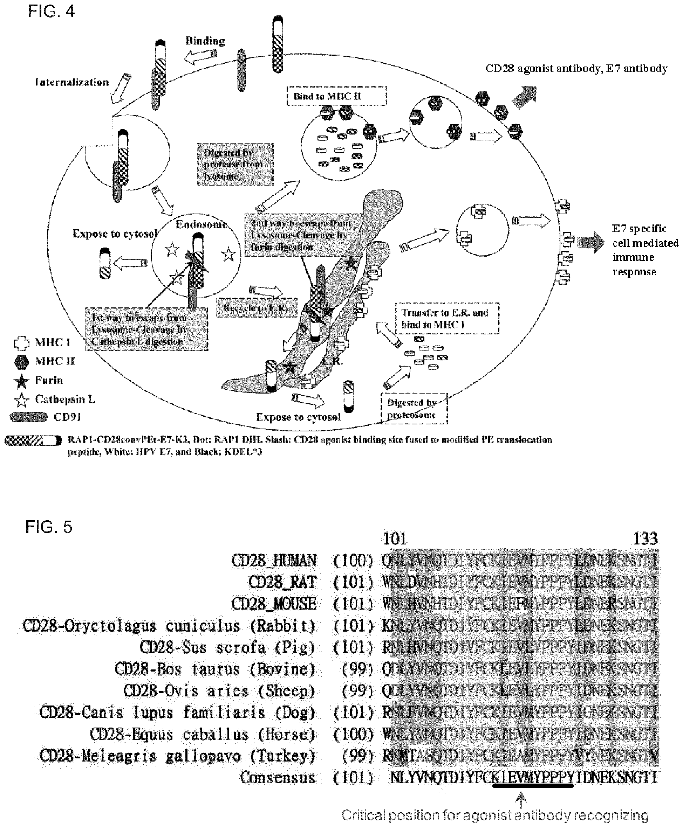

FIG. 4 is a schematic drawing illustrating the mechanisms of actions of T cell-sensitizing fusion proteins using the fusion protein RAP1-CD28convPEₜ-E7-K3 as an example. RAP1-CD28convPEₜ-E7-K3 comprises from N-terminus to the C-terminus: (1) the domain III of the full length RAP1 at the N-terminus, (2) CD28conv, (3) a linker, (4) a modified translocation peptide from Pseudomonas Exotoxin A, (5) a full length HPV type 16 E7 protein, and (6) the triple KDEL as an ER retention signal at the C-terminus. The HPV16 E7 protein is processed into epitopes within the cells of a subject immunized with the fusion protein. The RAP1-CD28convPEₜ-E7-K3 can elicit a better cytotoxic T-cell (CTL) response than the traditional vaccines that contain antigen only. RAP1-CD28convPEₜ-E7-K3 protein was designed to improve APC (such as dendritic cell) uptake efficiency and enhance HPV16 E7 antigen processing toward a proteasome pathway, then presented via MHC I complexes. The mechanism of action of HPV16 E7 protein-specific CTL immune response elicited by the vaccine RAP1-CD28convPEₜ-E7-K3 is illustrated in FIG. 4: (a) the vaccine binds to APC (such as dendritic cell) surface receptor (CD91) and internalized via endocytosis; (b1) RAP1-CD28convPEₜ-E7-K3 undergoes proteolytic hydrolysis by cathepsin L protease digestion at the site before the the translocation peptide PEₜ; (b2) or recycles to the E.R. and undergoes proteolytic hydrolysis by furin protease at the site before the translocation peptide PEₜ; (b3) At the meantime, RAP1-CD28conv was digested by lysosomal protease and the epitopes of CD28conv are then presented to the cell surface via MHC II and elicits CD28 agonist antibody production, which can pre-activate T cells; (c) The most important step is transmembrane translocation of the PEₜ-E7-K3 into the cytoplasmic compartment from the lysosome by translocation peptide (PEₜ); (d) the PEₜ-E7-K3 undergoes digestion via proteasome pathway, then the epitopes of E7 are presented by MHC I complex and elicit E7-specific cell mediated immune response.

FIG. 5 shows a sequence alignment of CD28 conserved regions from various species and the consensus sequence. The underlined sequence (KIEVMYPPPY; SEQ ID NO: 32, where X² is I, X⁴ is V, X⁵ is M) within the consensus sequence is a critical region for CD28 agonist antibody recognition and binding. This critical region sequence can be represented by K¹(I/L)²E³(V/F/A)⁴(M/L)⁵Y⁶P⁷P⁸P⁹Y¹⁰, in which only the fourth amino acid residue therein is species-specific and should be V in human, rat, porcine, bovine, sheep, dog and horse; F in mouse, and V in turkey. The critical region sequence may be represented as K¹(X)²E³(X)⁴(X)⁵Y⁶P⁷P⁸P⁹Y¹⁰ (SEQ ID NO: 32), wherein (X)² is I or L; (X)⁴ is V, F or A, (X)⁵ is M or L.

A PE translocation peptide may comprise the amino acid sequence consisting of SEQ ID NO: 3 or 20. For example, the amino acid sequence of a PE translocation peptide may be a.a. 280 - a.a. 313 (SEQ ID NO: 3), a.a. 268 - a.a. 313 (SEQ ID NO: 20), a.a. 253 - a.a. 313, or a.a. 253 - a.a. 364 (SEQ ID NO: 4) of PE. That is, the amino acid sequence of a PE translocation peptide may contain any region of the PE domain II (a.a. 253 to a.a. 364; SEQ ID NO: 4) as long as it comprises a.a. 280-a.a. 313 (SEQ ID NO: 3) essential fragment.

An antigen may be a pathogenic protein, polypeptide or peptide that is responsible for a disease caused by the pathogen, or is capable of inducing an immunological response in a host infected by the pathogen, or tumor-associated antigen (TAA) which is a polypeptide specifically expressed in tumor cells . The antigen may be selected from a pathogen or cancer cells including, but not limited to, Human Papillomavirus (HPV), PRRSV, HIV-1, flu virus, Dengue virus, Hepatitis C virus (HCV), Hepatitis B virus (HBV), Porcine Circovirus 2 (PCV2), non-small cell lung cancer, breast carcinoma, melanoma, lymphomas, colon carcinoma, hepatocellular carcinoma and any combination thereof. For example, HPV E7 protein (E7), HCV core protein (HCV core), HBV X protein (HBx) were selected as antigens for vaccine development. The antigen may be a fusion antigen from a fusion of two or more antigens selected from one or more pathogenic proteins. For example, a fusion antigen of PRRSV ORF6 and ORF5 fragments, or a fusion of antigenic proteins from PRRSV and PCV2 pathogens.

The function of an endoplasmic reticulum retention sequence is to assist translocation of an antigen from an endocytotic compartment into ER and retains it in the lumen. It comprises the sequence Lys Asp Glu Leu (KDEL) or RDEL. An ER sequence may comprise, or consists essentially of, or consist of, the sequence of KKDLRDELKDEL (SEQ ID NO: 16), KKDELRDELKDEL (SEQ ID NO: 17), KKDELRVELKDEL (SEQ ID NO: 18).

Receptor-associated protein (RAP1) with a molecular weight of 39 kDa is an ER resident protein and molecular chaperone for LDL receptor-related protein. It has a high binding affinity to CD91 (Kd∼ 3 nM) and is composed by three functional-similar domains.

The invention relates to the discovery of induction and enhancement of T cell mediated immune responses by fusion proteins according to the invention. Using RAP1-CD28convPEₜ-E7-K3 an example, the strategy of RAP1-CD28convPEₜ-E7-K3 vaccine is focused primarily on stimulating the production and activation of T cells that can recognize HPV16 infected cells expressing the target antigen E7. By delivering antigens to dendritic cells, it can generate antigen-specific CD8+ T cells and CD4+ T cells. Type 1-helper CD4+ T cells particularly are able to efficiently stimulate and augment the immune response of cytotoxic CD8+ T cells. Together, these two arms of the adaptive immune system have the specificity and potency to kill HPV16-infected cells or HPV16-associated tumor cells at multiple sites in the body without inflicting significant damage on normal tissues.

The term "subject" refers to a human or a non-human animal.

The term "treating" or "treatment" refers to administration of an effective amount of the fusion protein to a subject in need thereof, who has cancer or infection, or a symptom or predisposition toward such a disease, with the purpose of cure, alleviate, relieve, remedy, ameliorate, or prevent the disease, the symptoms of it, or the predisposition towards it. Such a subject can be identified by a health care professional based on results from any suitable diagnostic method.

The term "an effective amount" refers to the amount of an active compound that is required to confer a therapeutic effect on the treated subject. Effective doses will vary, as recognized by those skilled in the art, depending on rout of administration, excipient usage, and the possibility of co-usage with other therapeutic treatment.

Abbreviations: CD 28, Cluster of Differentiation 28; PE₄₀₇, Pseudomonas exotoxin A (PE) amino acid residues from position 1 to position 407.

### EXAMPLES

### EXAMPLE 1

### Constructions of Expression Vectors

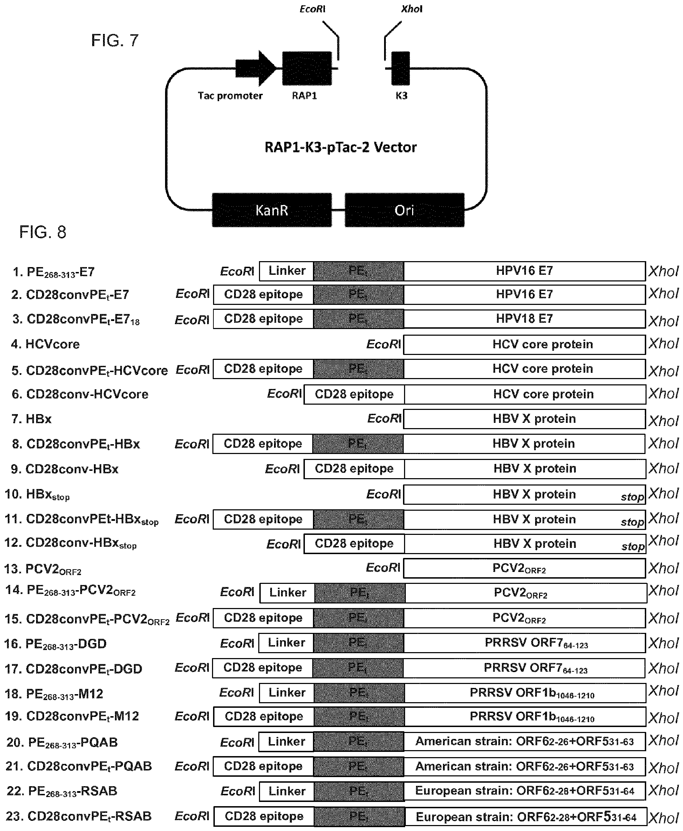

FIG. 7 shows the expression vector RAP1--K3 for construction of various fusion proteins. The vector comprises a RAP1 domain 3 (SEQ ID NO: 5), and an endoplasmic reticulum retention sequence (K3, or a KDEL signal; SEQ ID NO: 16, 17, 18, or 19). The translocation minimum essential peptide (PE₂₆₈₋₃₁₃; SEQ ID NO: 20) shown in FIG. 8 was obtained from PE (SEQ ID NO: 10) polypeptide sequence region from a.a. 268 to a.a. 313.

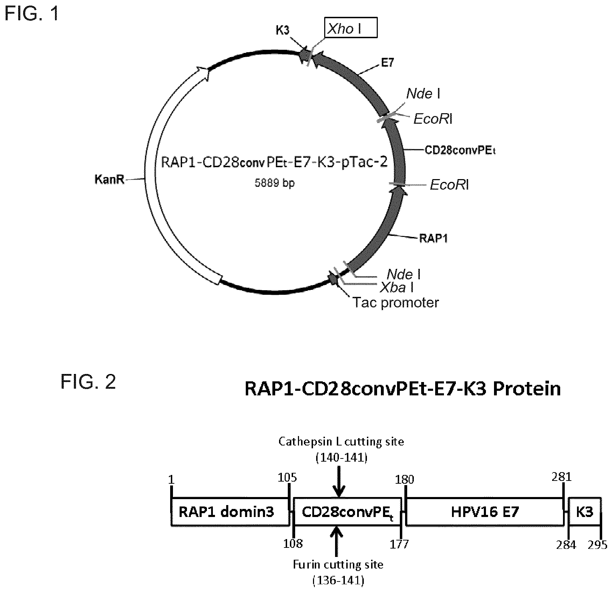

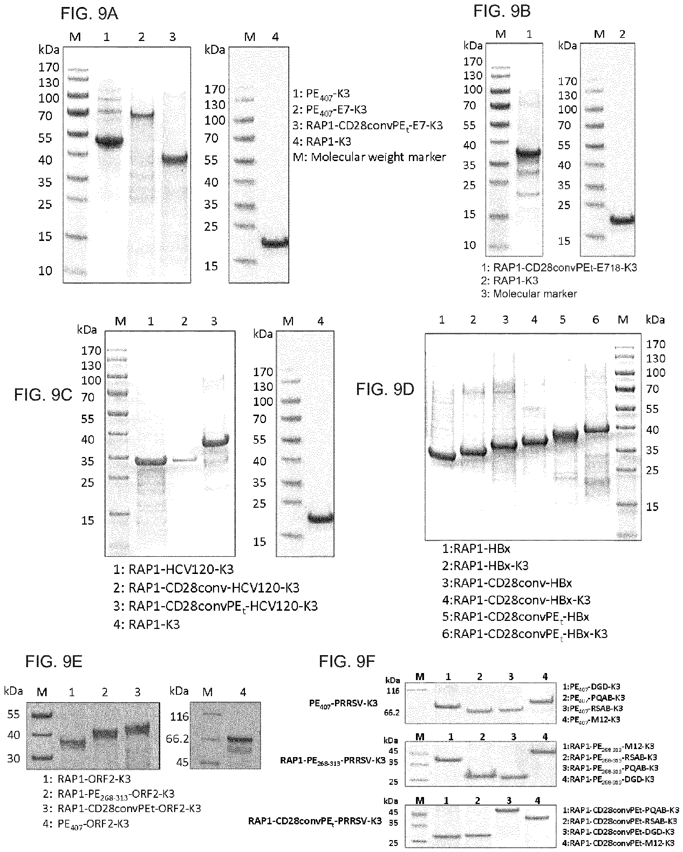

The plasmid RAP1-K3 (FIG. 7) was generated as follows: A DNA fragment encoding ^{NdeI}RAP1-^{(EcoRI, XhoI)}-K^{3XhoI} was synthesized by a PCR method and ligated into the plasmid pUC18 backbone with kanamycin resistance gene to obtain the plasmid RAP1-K3 (FIG. 7). DNA fragments encoding various fusion peptides were generated by PCR (FIG. 8) and inserted, respectively, into the plasmid RAP1-K3 (FIG. 7) to generate expression vectors for various fusion proteins (FIGs. 8 and 9A-F). FIG. 1 illustrates the expression vector for the fusion protein RAP1-CD28convPEₜ-E7-K3 (FIG. 1).

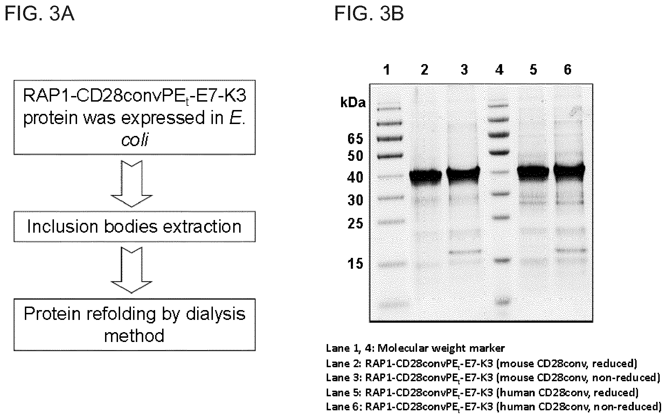

The fragment CD28convPEₜ contains cathepsin L and furin protease cutting sites. FIG. 3 shows an amino acid sequence map of the fusion protein RAP1-CD28convPEₜ-E7-K3 to illustrate the importance of the fragment CD28convPEₜ. The two arrows indicate cathepsin L and furin protease cutting sites, respectively. These two cutting sites not only serve as linkers to ligate CD28conv and PEₜ-E7 but also allow cutting of the fusion protein to release the fragment PEₜ-E7-K3 to the cytoplasm from the lysosome. Any other antigens of interest from various pathogens may replace E7 to fuse with CD28convPEₜ and the fusion product can then be inserted into the plasmid of FIG. 7 to make a variety of expression vectors for fusion proteins illustrated in FIG. 8.

The sequence of PE₂₆₈₋₃₁₃ is: pletftrhrqprgweqleqcgypvqrlvalylaarlswnqvdqvir (SEQ ID NO: 20). The whole sequence of CD28convPEₜ is as follows: tdiyfckiefmypppyldneksngtiihrarykrgweqleqcgypvqrlvalylaarlswnqvdqvirgs (SEQ ID NO: 30), the sequence underlined represents a linker sequence containing Cathepsin L and furin protease cutting sites.

### EXAMPLE 2

### Protein expression

*E. coli* BL21 cells harboring a protein expression vector were cultured in Luria Bertani broth containing 25 µg/ml kanamycin at 37°C. When the culture reached an early log phase, (A600=0.1 to 0.4), isopropyl-1-thio-β-D-galactopyranoside (IPTG) was added at a final concentration of 0.5 to 2 mM for induction. Cells were harvested after 4 hours IPTG induction and disrupted by sonication. The overexpressed protein-containing inclusion bodies were isolated and solubilized in 8M urea/TN buffer (8M urea, 50mM Tris, 50mM NaCl, pH 8.0).

RAP1 fusion proteins refolded easily and were not aggregated.

FIG. 3A is a flow chart illustrating that the fusion proteins RAP1-CD28PEₜ-E7-K3 (with mouse CD28conv or human CD28conv) were expressed and extracted from the inclusion bodies of *E. coli* cells). SDS-PAGE analyses indicated that the fusion proteins refolded well (FIG. 3B).

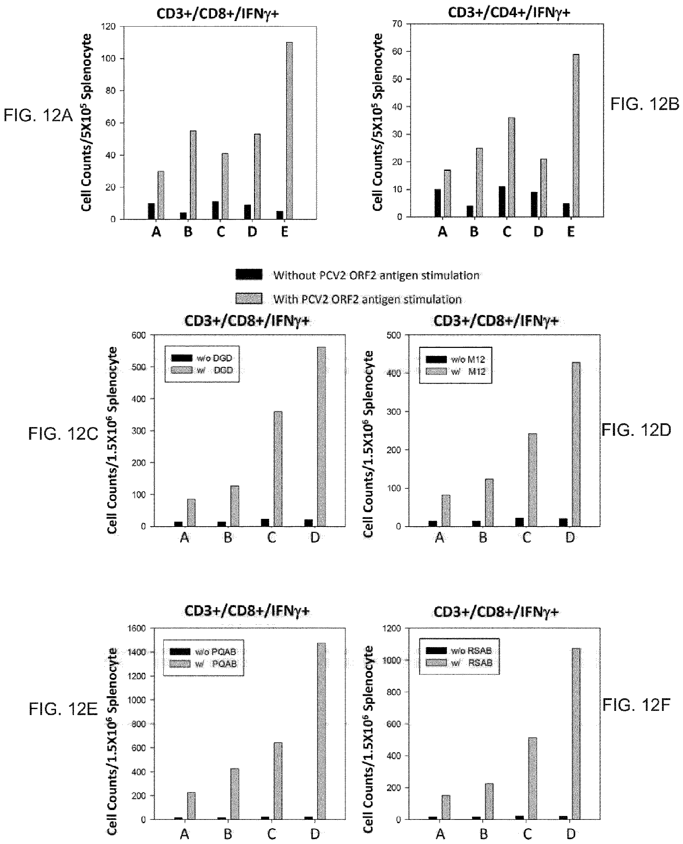

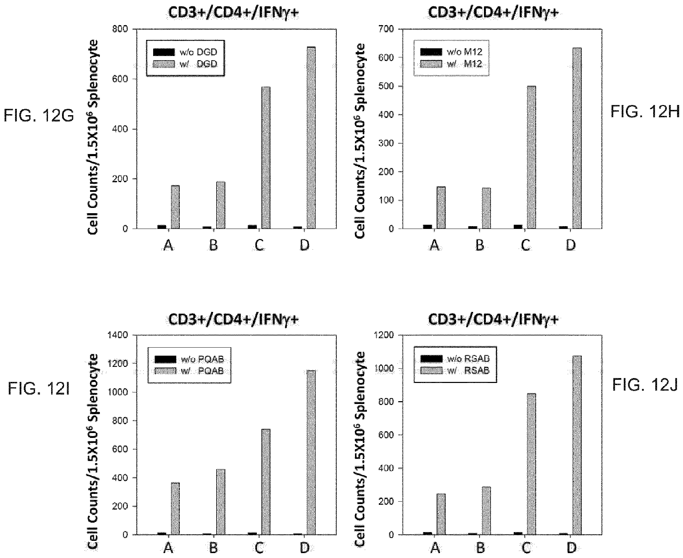

FIG. 8 illustrates a list of fusion proteins that were expressed using similar method described above: (1) RAP1-PE₂₆₈₋₃₁₃-E7-K3;(2) RAP1-CD28convPEₜ-E7-K3;(3) RAP1-CD28PEₜ-E7₁₈-K3;(4) RAP1-HCVcore-K3; (5) RAP1-CD28conv-HCVcore-K3; (6) RAP1-CD28convPEₜHCVcore-K3; (7) RAP1-HBx; (8) RAP1-HBx-K3; (9) RAP1-CD28conv-HBx; (10) RAP1-CD28conv-HBx-K3; (11) RAP1-CD28convPEₜ-HBx; (12) RAP1-CD28convPEₜ-HBx-K3; (13) RAP1-PCV2_{ORF2}-K3; (14) RAP1- PE₂₆₈₋₃₁₃-PCV2_{ORF2}-K3; (15) RAP1-CD28convPEₜ-PCV2_{ORF2}-K3; (16) RAP1-PE₂₆₈₋₃₁₃-DGD-K3; (17) RAP1-PE₂₆₈₋₃₁₃-M12-K3; (18) RAP1-PE₂₆₈₋₃₁₃-PQAB-K3; (19) RAP1-PE₂₆₈₋₃₁₃-RSAB-K3; (20) RAP1-CD28convPEₜ-DGD-K3; (21) RAP1-CD28convPEₜ-M12-K3; (22) RAP1-CD28convPEₜ-PQAB-K3; (23) RAP1-CD28convPEₜ-RSAB-K3. These fusion proteins were refolded using the same method described above. The results of SDS-PAGE analyses indicated these fusion proteins al refolded well and were thus used for preparing vaccines (FIGs. 12A-F). The term "K3" stands for the amino acid seqeunce KDELKDELKDEL (SEQ ID NO: 19).

### EXAMPLE 3

### RAP1-CD28convPEₜ-E7-K3 inhibits growth of Tumors induced by human papilloma virus (HPV) type 16 E7 protein and increases survival rate

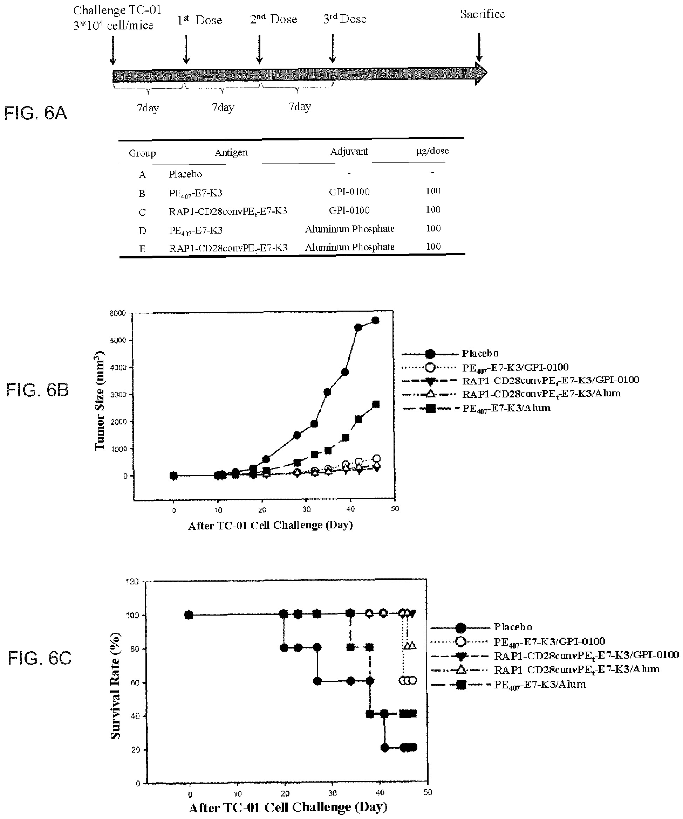

The effects of the fusion proteins PE₄₀₇-E7-K3 and RAP1-CD28convPEₜE7-K3 with or without an immune potentiator on tumor size and survival rate were examined. Mice were challenged with a higher dose of TC-01 cells (3x10⁴) via s.c. injection. Seven days after the challenge, mice were vaccinated via s.c. with placebo, PE₄₀₇-E7-K3 (100 µg/dose) or RAP1-CD28convPEₜ-E7-K3 (100 µg/dose) with the immune potentiator GPI-0100 or the protein absorbent aluminum phosphate once per week for 3 weeks (FIG. 6A). GPI-0100 is a Th1/CTL stimulating adjuvant (immune potentiator). The size of the tumors and the survival rate in each group were recorded (FIGs. 9B-C). When combined with the adjuvant GPI-0100, both PE₄₀₇-E7-K3 and RAP1-CD28convPEₜ-E7-K3 suppressed the tumor growth. Unexpectedly, it was discovered that the effect of RAP1-CD28convPEₜ-E7-K3 in inhibiting tumor growth was not dependent on the adjuvant. When combined with the absorbent aluminum phosphate rather than with the adjuvant GPI-0100, RAP1-CD28convPEₜ-E7-K3 could still significantly suppress the tumor growth with the same potency as that when combined with the immune potentiator GPI-0100 (FIG. 6B, unfilled triangle v. filed reverse triangle).

In contrast, the potency of PE₄₀₇-E7-K3 in suppressing the tumor growth depended on the adjuvant. When combined with the absorbent aluminum phosphate, PE₄₀₇-E7-K3 became less potent than that when combined with the immune potentiator GPI-0100 (FIG. 6B solid square v. unfilled circle).

On the other hand, mice administrated with RAP1-CD28convPEₜ-E7-K3 in combination with the immune potentiator GPI-0100 or the absorbent aluminum phosphate had a better survival rate than the groups vaccinated with PE₄₀₇-E7-K3 in combination with GPI-0100 or aluminum phosphate (FIG. 6C). This indicated that RAP1-CD28convPEₜ-E7-K3 could elicit Th1/CTL immune responses even without the immune potentiator GPI-0100. The results also indicated that the fusion protein RAP1-CD28convPEₜ-E7-K3 was superior to PE₄₀₇-E7-K3 as a vaccine for increasing the survival rate of the animals.

### EXAMPLE 4

### Immunogenicity assays

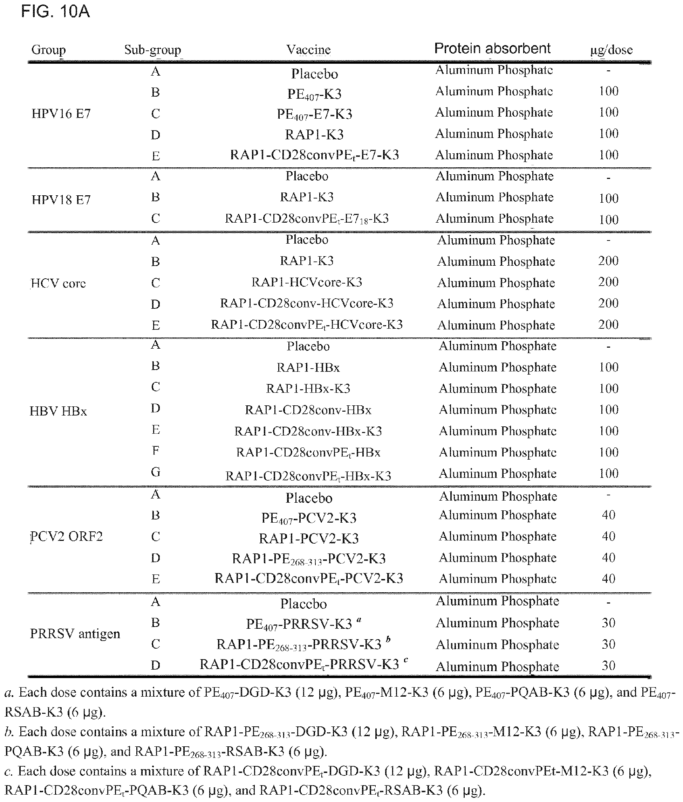

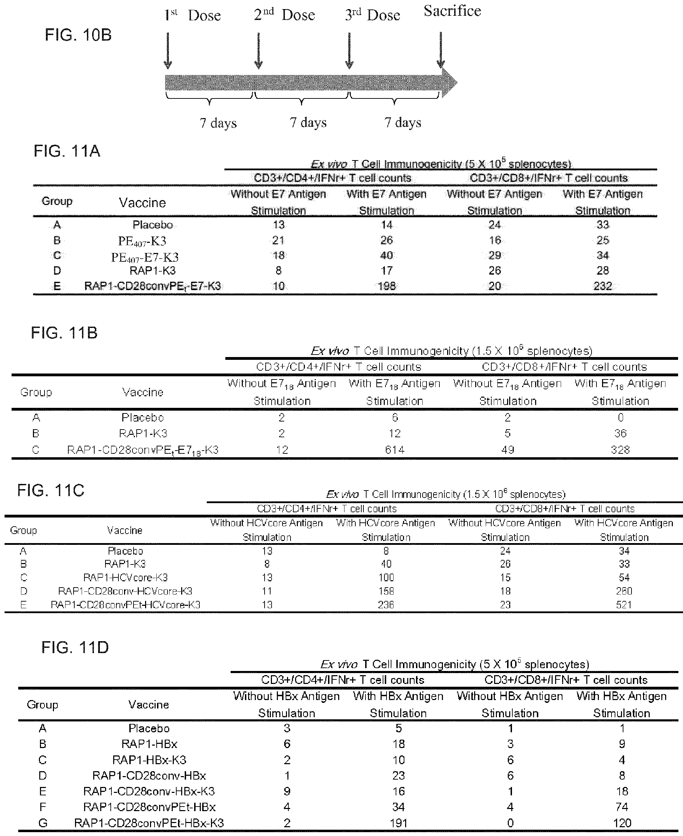

The immunogenicities of various vaccines were tested. Briefly, mice were divided into the following groups: HPV16 E7, HPV 18 E7, HCV core, HBV HBx, PCV2 ORF2 and PRRSV (FIG. 10A). Each group was further divided into subgroups, and each subgroup was injected with either a placebo or a vaccine designed to target toward a certain antigen, or certain antigens, of a pathogen via s.c. once per week for 3 weeks (FIG. 10B). Except the vaccines targeted to PRRSV, each vaccine was composed of a single fusion protein and the absorbent aluminum phosphate, in which the single fusion protein contained at least an antigen of a pathogen. The antigen was either a full-length protein from a pathogen, or a non-full-length protein that contained at least one epitope of an antigen of a pathogen, or was a fusion peptide of two or more antigens, in which each of the antigens was selected from different proteins of a pathogen.

The immunization schedule, vaccines and dose are illustrated in FIGs. 10A-B. Briefly, mice were vaccinated once per week for 3 weeks with vaccines listed in FIG. 10A. All mice were sacrificed 7 days after the last immunization, and the spleens were harvested. Splenocytes were isolated and cultured in 6-well plate (2x10⁷ cells/2ml/well) with 10 µg/ml of respective recombinant antigens of pathogens to stimulate the splenocytes in the presence of 1 µg/ml GolgiPlug (BD Pharmingen, San Diego, CA) at 37°C for 16 hr.

The stimulated splenocytes were washed with FACScan buffer and the cell surface markers CD8a, CD4, and CD3 were stained with phycoerythrin-conjugated monoclonal rat anti-mouse CD8a, AF700-conjugated monoclonal rat anti-mouse CD4 and AF647-conjugated monoclonal rat anti-mouse CD3 antibodies. The cells were then permeabilized and fixed by Cytofix/Cytoperm kit according to the manufacturer's instructions (BD Pharmingen). Intracellular IFN-γ was stained with AF488-conjugated rat anti-mouse IFN-γ to measure the immune response and cytokine levels. Flow cytometry analyses were performed using Gallios flow cytometry with Kaluza analysis software (Beckman Coulter).

The following PRRSV vaccines were tested for immunogenicities: PE₄₀₇-PRRSV-K3, RAP1-PE₂₆₈₋₃₁₃-PRRSV-K3 or RAP1-CD28convPEₜ-PRRSV-K3 vaccine. Each vaccine contained a mixture of four different fusion proteins, and each fusion protein contained a different antigen that is selected from the group consisting of DGD, M12, PQAB and RSAB (FIG. 10A). Vaccination of mice and stimulations of splenocytes were performed using similar method as described above. Briefly, all mice were sacrificed 7 days after the last immunization, and spleens were harvested. The splenocytes were isolated and cultured in 6-well plate (2x10⁷ cells/2ml/well) with 10 µg/ml of the recombinant DGD, M12, PQAB or RSAB antigens, separately, to stimulate the splenocytes in the presence of 1 µg/ml GolgiPlug (BD Pharmingen, San Diego, CA) at 37°C for 16 hr.

The amino acid sequence of "DGD" (SEQ ID NO: 26) is as follows: **RHHFTPSERQLCLSSIQTAFNQGAGTCILSDSGRISYTVEFSLPTHHTVRLIRVTAPPS** **A**LDQVIRNALASPGSGGDLGEAIREQPEQARLALTLAAAESERFVRQGTGNDEAGAANADVVS LTCPVAAGECAGPADSGDALLERNYPTGAEFLGDGGDV**RHHFTPSERQLCLSSIQTAFNQGA GTCILSDSGRISYTVEFSLPTHHTVRLIRVTAPPSA.** DGD represents a fusion antigen of PRRSV ORF7 a.a. 64 - a.a. 123 (boldface), linker (underlined) and ORF7 a. a. 64- a.a. 123 (boldface).

The term "M12" represents a antigen of PRRSV ORFlb a.a. 1046 - a.a. 1210. Its amino acid sequence (SEQ ID NO: 27) is as
follows: NNKECTVAQALGNGDKFRATDKRVVDSLRAICADLEGSSSPLPKVAHNLGFYFSPDLT QFAKLPIELAPHWPVVSTQNNEKWPDRLVASLRPLDKYSRACIGAGYMVGPSVFLGTPGVVSY YLTKFVKGEAQVLPETVFSTGRIEVDCREYLDDREREVAASLPH.

The amino acid sequence of "PQAB" (SEQ ID NO: 28) is as follows: GSSLDDFCYDSTAPQKVLLAFSITYASNDSSSHLQLIYNLTLCELNGTDWLANKFDWA. PQAB represents a fusion antigen of PRRSV American strain ORF6 a.a. 2- a.a. 26 and ORF5 a.a. 31- a.a. 63 (underlined).

The amino acid sequence of RSAB is MGSLDDFCNDSTAAQKLVLAFSITYTPIFVAGGSSSTYQYIYNLTICELNGTDWLSNHFDWA (SEQ ID NO: 29). The term "RSAB" represents a fusion antigen of PRRSV European strain ORF6 a.a. 2-28 and ORF5 a.a. 31-64 (underlined).

### EXAMPLE 5

The fragment of RAP1 domain 3 of the fusion protein RAP1-CD28convPEₜ-E7-K3 is replaced by A2M minimum (SEQ ID NO: 6), HIV-Tat minimum (SEQ ID NO: 7) or HSPs minimum (SEQ ID NO: 8) to generate the fusion proteins A2M- CD28convPEₜ-E7-K3, Tat-CD28convPEₜ-E7-K3 and HSP-CD28convPEₜ-E7-K3 vaccines, respectively. The TC-1 tumor suppression activity and cell mediated immune responses enhanced by these vaccines are examined using similar methods as described above. Table 1 shows SEQ ID NOs. of the components of various fusion proteins. Table 2 shows the fusion proteins tested for the effects on T cell-mediated immune responses in animals and the sequences of antigens.

**Table 1**

| Component | SEQ ID NO: | Length (residues) |
|---|---|---|
| hCD28 Core | 1 | 28 |
| TDIYFCKIEVMYPPPYLDNEKSNGTIIH | | |
| hCD28 Maximum | 2 | 53 |
| | | |
| PEₜ Core (PE translocation domain core; a.a. 280- a.a. 313 of PE) | 3 | 34 |
| PEₜ Maximum (translocation domain maxi, a.a. 253 - a.a. 364 of PE) | 4 | 112 |
| RAP1 Minimum (domain III of RAP 1) | 5 | 104 |
| A2M Minimum | 6 | 153 |
| HIV-Tat Minimum | 7 | 24 |
| HSPs Minimum, Heat shock 70 kDa protein (HSPs; Homo sapiens) | 8 | 641 |
| Minimum Pseudomonas exotoxin A (PE) binding domain Ia (an APC-binding domain, a.a. 1- a.a. 252 of PE) | 9 | 252 |
| Linker RXRXKR, ), in which "X" is any amino acid residue. | 15 | 6 |
| Full length PE (Exotoxin A mature form, *Pseudomonas aeruginosa*) | 10 | 613 |
| Full length RAP1 *(Homo sapiens* low density lipoprotein receptor-related protein associated protein 1, LRPAP1); Domain 1: a.a. 1- a.a. 112; domain 2: a.a. 113 - a.a. 218; domain 3: a.a. 219 - a.a. 323. | 11 | 323 |
| Full- length A2M (*Homo sapiens* alpha-2-macroglobulin receptor-associated protein precursor) | 12 | 357 |
| HIV-Tat (Human immunodeficiency virus 1) | 13 | 101 |
| KDEL | 14 | 4 |
| KKDLRDELKDEL | 16 | 12 |
| KKDELRDELKDEL | 17 | 13 |
| KKDELRVELKDEL | 18 | 13 |
| KDELKDELKDEL | 19 | 12 |
| PE₂₆₈₋₃₁₃ | 20 | |
| | | 46 |
| CD28convPEₜ | 30 | 68 |
| | | |
| wherein (X)⁸ is I or L; (X)¹⁰ is V, F or A, (X)¹¹ is M or L, X¹⁷ is L or I, (X)^{30, 32} is any amino acid residue. | | |
| CD28 consensus sequence | 31 | 28 |
| | | |
| , wherein (X)⁸ is I or L; (X)¹⁰ is V, F or A, (X)¹¹ is M or L, X¹⁷ is L or I. | | |
| CD28 critical region | 32 | 10 |
| K¹(X)²E³(X)⁴(X)⁵Y⁶P⁷P⁸P⁹Y¹⁰, wherein (X)² is I or L; (X)4 is V, F or A, (X)⁵ is M or L. | | |

**Table 2**

| Fusion protein name | Antigen Name | Antigen SEQ ID NO: |
|---|---|---|
| RAP1-CD28convPEₜ-E7-K3 | HPV16 E7 (full length) | 21 |
| RAP1-CD28convPEₜ-E7₁₈-K3 | HPV18 E7 (full length) | 22 |
| RAP1-CD28convPEₜ-HCVc-K3 | HCV core protein (full length) | 23 |
| RAP1-CD28convPEₜ-HBx-K3 | HBV X protein (full length) | 24 |
| RAP1-CD28convPEₜ-PCV2-K3 | PCV2 ORF2 (a fragment of ORF2) | 25 |
| RAP1-CD28convPEₜ-DGD-K3 | PRRSV nucleocapsid (a fusion antigen: ORF7 a.a. 64 - a.a. 123, linker and ORF7 a.a. 64- a.a. 123) | 26 |
| RAP1-CD28convPEₜ-M12-K3 | PRRSV RNA-dependent RNA polymerase (ORF1b a.a.1046-a.a. 1210) | 27 |
| RAP1-CD28convPEₜ-PQAB-K3 | PRRSV American strain: a fusion antigen of ORF6 (a.a. 2-a.a. 26) and ORF5 (a.a. 31- a.a. 63) | 28 |
| RAP1-CD28convPEₜ-RSAB-K3 | PRRSV European strain: a fusion antigen of ORF6 (a.a. 2-a.a. 28) and ORF5 (a.a. 31-a.a. 64) | 29 |

In the immunogenicity assays, antigen-specific cell-mediated immune responses induced by various vaccines were evaluated by measuring the numbers of CD3+/CD4+/IFNy+ and CD3+/CD8+/IFNy+ T cells in the splenocytes (FIGs. 11A-D and FIGs. 12A-J). The results indicated that the vaccine RAP1-CD28convPEₜ-antigen-K3 can induce strong T cell responses (FIGs. 11A-D and FIGs. 12A-J). FIG. 11B shows the CD3+/CD4+/IFNy+ T cell number and the CD3+/CD8+/IFNy+ T cell number elicited by CD28convPEₜ-E7₁₈-K3 were about 50 times and greater than 9 times of RAP1-K3, respectively.

The vaccine RAP1-CD28convPEₜ-antigen-K3 is superior to PE₄₀₇-antigen-K3 in eliciting T cell-mediated immunogenicity. For example, FIG. 11A illustrates the CD3+/CD4+/IFNy+ T cell number and the CD3+/CD8+/IFNy+ T cell number elicited by CD28convPEₜ-E7₁₆-K3 were about 5 times and 7 times of PE₄₀₇-E7₁₆-K3, respectively. This indicates that the vaccine RAP1-CD28convPEₜ-E7-K3 had a better cell-mediated immunogenicity than PE₄₀₇-E7-K3.

A fusion protein comprising RAP1 domain III, the sensitizing signal CD28conv alone without the translocation peptide PEₜ, antigen and an ER retention signal is sufficient in eliciting a strong antigen-specific T cell mediated immune responses when the antigen chosen comprises ten or greater than 10 epitopes. FIG. 11C illustrates the vaccine RAP1-CD28conv-HCVcore-K3 elicited T cell responses with the numbers of CD3+/CD4+/IFNy+ and of CD3+/CD8+/IFNy+ T cells being 20 times and 7.6 times of the placebo group, respectively. The antigen HCVcore contains 11well-known MHC I epitopes.

It was unexpected that the ER retention signal is not essential for the fusion protein of the invention to elicit a strong cell-mediated immunogenicity. In other words, without the ER retention sequence, the fusion protein of the invention can still elicit strong T-cell responses. FIG. 11D illustrates that the numbers of CD3+/CD4+/IFNy+ and CD3+/CD8+/IFNy+ T cells elicited by RAP1-CD28convPEₜ-HBx (without the ER retentions signal K3) were 7 times and 74 times of the placebo group.

In contrast, U.S. Patent Numbers 7378100B2 and 7335361 show that the ER retention signal K3 is indispensable for PE-related fusion proteins (PE₄₀₇-antigen-K3) to elicit T cell responses.

FIGs. 12A-B illustrate the vaccine RAP1- CD28convPEₜ-PCV2-K3 and FIG. 12C-J illustrate the vaccine RAP1-CD28convPEₜ-PRRSV-K3 elicited greatest CD3+/CD4+/IFNy+ and CD3+/CD8+/IFNy+ T cell counts among the vaccines (FIG. 10A) tested.

## Claims

1. A fusion protein comprising:
(a) an antigen-presenting cell (APC)-binding domain or a CD91 receptor-binding domain, located at the N-terminus of the fusion protein, wherein the APC-binding domain or CD91 receptor-binding domain is selected from the group consisting of receptor-associated protein-1 (RAP1) domain III, alpha-2-macroglobulin receptor-associated protein (A2M), HIV-Tat, heat shock 70kDa protein, and Pseudomonas exotoxin A binding domain la;
(b) a protein transduction domain, located at the C-terminus of the APC-binding domain or the CD91 receptor-binding domain, the protein transduction domain is a fusion polypeptide comprising a T cell sensitizing signal-transducing peptide, a linker, and a translocation peptide, wherein:
(1) the T cell sensitizing signal-transducing peptide is located at the N-terminus of the fusion polypeptide;
(2) the linker comprises SEQ ID NO: 15, linking the T cell sensitizing signal-transducing peptide and the translocation peptide; and
(3) the translocation peptide has 34-112 amino acid residues in length and comprises an amino acid sequence consisting of SEQ ID NO: 3, 20 or 4; and
(c) an antigen of a pathogen, located at the C-terminus of the protein transduction domain;
wherein:
the T cell-sensitizing signal-transducing peptide has 28-53 amino acid residues in length and comprises the amino acid sequence of SEQ ID NO: 31, in which Xaa⁸ is I or L; Xaa¹⁰ is V, F or A, Xaa¹¹ is M or L, Xaa¹⁷ is L or I.

2. The fusion protein of claim 1, wherein the APC-binding domain or the CD91 receptor-binding domain is a polypeptide comprising an amino acid sequence that is at least 90% identical to the sequence selected from the group consisting of SEQ ID NOs: 5, 9, 6, 7, and 8.

3. The fusion protein of claim 1, further comprising an endoplasmic reticulum retention sequence located at the C-terminus of the fusion protein.

4. The fusion protein of claim 3, wherein the endoplasmic reticulum retention sequence comprises the amino acid sequence of Lys -Asp-Glu-Leu (SEQ ID NO: 14).

5. The fusion protein of claim 1, wherein the fusion protein is free of an endoplasmic reticulum retention sequence at C-terminus thereof if the antigen contains 10 or more epitopes.

6. The fusion protein of claim 1, wherein the protein transduction domain comprises the sequence of SEQ ID NO: 30.

7. The fusion protein of claim 1, wherein the APC-binding domain or the CD91 receptor-binding domain is a polypeptide comprising an amino acid sequence selected from the group consisting of SEQ ID NOs: 5, 9, 6, 7, and 8.

8. The fusion protein of claim 1, wherein the T cell sensitizing signal-transducing peptide comprises an amino acid sequence selected from the group consisting of SEQ ID NO: 1 and 2.

9. The fusion protein of claim 1, wherein the translocation peptide comprises the amino acid sequence of SEQ ID NO: 3.

10. A fusion protein as claimed in claim 1 for use in inducing enhanced pathogen antigen-specific T cell responses, killing a disease cell that presents an antigen via class I MHC molecules on the cell membrane of the disease cell, and/or preventing, treating infection caused by a pathogen, and/or minimizing symptoms caused by the infection.

11. The fusion protein for use as claimed in claim 10, wherein the disease cell is a cancer cell.

## Patentansprüche

1. Fusionsprotein, umfassend:
(a) eine Antigenpräsentierende Zellen (APC)-Bindungsdomäne oder eine CD91-Rezeptor-Bindungsdomäne, die an dem N-Terminus des Fusionsproteins angeordnet ist, wobei die APC-Bindungsdomäne oder die CD91-Rezeptor-Bindungsdomäne ausgewählt ist aus der Gruppe bestehend aus Rezeptor-assoziiertem Protein-1 (RAP1) Domäne III, Alpha-2-Makroglobulin Rezeptor-assoziiertem Protein (A2M), HIV-Tat, Hitzeschockprotein 70kDa und Pseudomonas Exotoxin A Bindungsdomäne Ia;
(b) eine Protein-Transduktionsdomäne , die an dem C-Terminus der APC-Bindungsdomäne oder die CD91-Rezeptor-Bindungsdomäne angeordnet ist, wobei die Protein-Transduktionsdomäne ein Fusionspolypeptid ist, das eine T-Zellen-Sensibilisierungssignal übermittelndes Peptid, einen Linker und ein Translokationspeptid umfasst, wobei:
(1) sich das T-Zellen-Sensibilisierungssignal übermittelnde Peptid an dem N-Terminus des Fusionspolypeptids befindet;
(2) der Linker SEQ ID NO: 15 umfasst und das T-Zellen-Sensibilisierungssignal und das Translokationspeptid verbindet; und
(3) das Translokationspeptid eine Länge von 34 bis 112 Aminosäureresten aufweist und eine Aminosäuresequenz umfasst, die aus der SEQ ID NO: 3, 20 oder 4 besteht; und
(c) ein Antigen eines Pathogens, das an dem C-Terminus der Protein-Transduktionsdomäne angeordnet ist;
wobei:
das T-Zellen-Sensibilisierungssignal übermittelnde Peptid eine Länge von 28 bis 53 Aminosäureresten aufweist und die Aminosäuresequenz der SEQ ID NO: 31 umfasst, wobei Xaa⁸ I oder L ist; Xaa¹⁰ V, F oder A ist, Xaa¹¹ M oder L ist, Xaa¹⁷ L oder I ist.

2. Fusionsprotein nach Anspruch 1, wobei die APC-Bindungsdomäne oder die CD91-Rezeptor-Bindungsdomäne ein Polypeptid ist, das eine Aminosäuresequenz umfasst, die zu mindestens 90% identisch ist mit der Sequenz, die ausgewählt ist aus der Gruppe bestehend aus den SEQ ID NO: 5, 9, 6, 7 und 8.

3. Fusionsprotein nach Anspruch 1, ferner umfassend eine endoplasmatische Retikulum-Retentionssequenz an dem C-Terminus des Fusionsproteins.

4. Fusionsprotein nach Anspruch 3, wobei die endoplasmatische Retikulum-Retentionssequenz die Aminosäuresequenz von Lys-Asp-Glu-Leu (SEQ ID NO: 14) umfasst.

5. Fusionsprotein nach Anspruch 1, wobei das Fusionsprotein an dessen C-Terminus keine endoplasmatische Retikulum-Retentionssequenz aufweist, wenn das Antigen 10 oder mehr Epitope enthält.

6. Fusionsprotein nach Anspruch 1, wobei die Protein-Transduktionsdomäne die Sequenz der SEQ ID NO: 30 umfasst.

7. Fusionsprotein nach Anspruch 1, wobei die APC-Bindungsdomäne oder die CD91-Rezeptor-Bindungsdomäne ein Polypeptid ist, das eine Aminosäuresequenz umfasst, die ausgewählt ist aus der Gruppe bestehend aus den SEQ ID NO: 5, 9, 6, 7 und 8.

8. Fusionsprotein nach Anspruch 1, wobei das T-Zellen-Sensibilisierungssignal übermittelnde Peptid eine Aminosäuresequenz umfasst, die ausgewählt ist aus der Gruppe bestehend aus den SEQ ID NO: 1 und 2.

9. Fusionsprotein nach Anspruch 1, wobei das Translokationspeptid die Aminosäuresequenz der SEQ ID NO: 3 umfasst.

10. Fusionsprotein nach Anspruch 1 zur Verwendung in der Induktion verbesserter pathogener antigenspezifischer T-Zellen-Reaktionen, zum Abtöten einer Krankheitszelle, die ein Antigen präsentiert, über Klasse I MHC-Moleküle an der Zellmembran der Krankheitszelle, und/oder für eine Prävention, Behandlung einer durch ein Pathogen bewirkten Infektion, und/oder zur Minimierung von durch die Infektion bewirkten Symptomen.

11. Fusionsprotein zur Verwendung nach Anspruch 10, wobei die Krankheitszelle eine Krebszelle ist.

## Revendications

1. Protéine hybride comprenant :
(a) un domaine de liaison à la cellule présentatrice d'antigène (APC) ou un domaine de liaison au récepteur CD91, situé à l'extrémité N-terminale de la protéine hybride, le domaine de liaison à l'APC ou le domaine de liaison au récepteur CD91 étant choisi dans le groupe constitué par le domaine III de la protéine associée au récepteur-1 (RAP1), la protéine associée au récepteur alpha-2-macroglobuline (A2M), VIH-Tat, protéine 70kDa du choc thermique et domaine de liaison à la Pseudomonas Exotoxin A ;
(b) un domaine de transduction protéique, situé à l'extrémité C-terminale du domaine de liaison à l'APC ou du domaine de liaison au récepteur CD91, le domaine de transduction protéique étant un polypeptide hybride comprenant un peptide de transduction de signal de sensibilisation de lymphocytes T, un lieur et un peptide de translocation,
(1) le peptide de transduction de signal de sensibilisation de lymphocytes T étant situé à l'extrémité N-terminale du polypeptide hybride ;
(2) le lieur comprenant SEQ ID No : 15, liant le peptide de transduction de signal de sensibilisation de lymphocytes T et le peptide de translocation ; et
(3) le peptide de translocation ayant une longueur de 34 à 112 résidus d'acides aminés et comprenant une séquence d'acides aminés consistant en SEQ ID No : 3, 20 ou 4 ; et
(c) un antigène d'un pathogène, situé à l'extrémité C-terminale du domaine de transduction protéique ;
le peptide de transduction de signal de sensibilisation de lymphocytes T ayant une longueur de 28 à 53 résidus d'acides aminés et comprenant la séquence d'acides aminés de SEQ ID No : 31, Xaa⁸ étant I ou L; Xaa¹⁰ étant V, F ou A, Xaa¹¹ étant M ou L, Xaa¹⁷ étant L ou I.

2. Protéine hybride selon la revendication 1, le domaine de liaison à l'APC ou le domaine de liaison au récepteur CD91 étant un polypeptide comprenant une séquence d'acides aminés qui est identique à au moins 90 % à la séquence choisie dans le groupe constitué par SEQ ID No : 5, 9, 6, 7, et 8.

3. Protéine hybride selon la revendication 1, comprenant en outre une séquence de rétention du réticulum endoplasmique située à l'extrémité C-terminale de la protéine hybride.

4. Protéine hybride selon la revendication 3, la séquence de rétention du réticulum endoplasmique comprenant la séquence d'acides aminés de Lys-Asp-Glu-Leu (SEQ ID No : 14).

5. Protéine hybride selon la revendication 1, la protéine hybride étant exempte d'une séquence de rétention du réticulum endoplasmique à son extrémité C-terminale si l'antigène contient 10 épitopes ou plus.

6. Protéine hybride selon la revendication 1, le domaine de transduction protéique comprenant la séquence de SEQ ID No : 30.

7. Protéine hybride selon la revendication 1, le domaine de liaison à l'APC ou le domaine de liaison au récepteur CD91 étant un polypeptide comprenant une séquence d'acides aminés choisie dans le groupe constitué par SEQ ID No : 5, 9, 6, 7, et 8.

8. Protéine hybride selon la revendication 1, le peptide de transduction de signal de sensibilisation de lymphocytes T comprenant une séquence d'acides aminés choisie dans le groupe constitué de SEQ ID No : 1 et 2.

9. Protéine hybride selon la revendication 1, le peptide de translocation comprenant la séquence d'acides aminés de SEQ ID No : 3.

10. Protéine hybride selon la revendication 1 destinée à être utilisée dans l'induction de réponses améliorées des lymphocytes T spécifiques à l'antigène pathogène, la destruction d'une cellule malade qui présente un antigène via des molécules IMHC de classe I sur la membrane cellulaire de la cellule malade, et/ou la prévention, le traitement d'une infection causée par un pathogène, et/ou la réduction des symptômes causés par l'infection.

11. Protéine hybride destinée à être utilisée selon la revendication 10, la cellule malade étant une cellule cancéreuse.
